# EUROPEAN PATENT APPLICATION

(11) **EP 4 382 120 A1**
(43) Date of publication of application: **12.06.2024**
(21) Application number: 22306787.7
(22) Date of filing: 05.12.2022
(51) Int. Cl.: A61K 39/00, C07K 16/28, A61P 35/04

(54) **ANTI-SLC1A4 MONOCLONAL ANTIBODIES AND USES THEREOF**

(71) Applicant: Institut Regional du Cancer de Montpellier, 34090 Montpellier (FR); INSERM (INSTITUT NATIONAL DE LA SANTE ET DE LA RECHERCHE MEDICALE), 75013 Paris (FR); UNIVERSITE DE MONTPELLIER, 34090 Montpellier (FR)
(72) Inventor: LINARES, Laetitia, 34160 SAINT JEAN DE CORNIES (FR); BOUILLIN, Alix, 34980 SAINT GELY DU FESC (FR); GAYTE, Laurie, 34160 GALARGUES (FR); CHENTOUF, Myriam, 34790 GRABELS (FR); RISCAL, Romain, 34130 LANSARGUES (FR); CARRERE, Sébastien, 34090 MONTPELLIER (FR); NEIVEYANS, Madeline, 34160 CASTRIES (FR)
(74) Representative: Cabinet Nony

(57) **Abstract**

The present disclosure relates to monoclonal antibodies directed against the external loop of SLC1A4, which monoclonal antibodies are preferably human monoclonal antibodies.

The present disclosure also relates to the therapeutic use of the said monoclonal antibodies, especially for treating serine-dependent cancers, particular SLC1A4-expressing cancers. It also relates to the *in vitro* and the *in vivo* use of the anti-SLC1A4 monoclonal antibodies disclosed therein for diagnosis purpose.

## Description

### FIELD OF THE DISCLOSURE

The present disclosure relates to the field of diagnostic and treatment of serine-dependent cancers, and especially of SLC1A4-expressing cancers. It relates to monoclonal antibodies directed against human SLC1A4 and uses thereof in diagnostic and therapeutic methods.

### BACKGROUND OF THE DISCLOSURE

Cancer refers to a group of diseases characterized by the development of abnormal cells that divide uncontrollably and can infiltrate and destroy normal body tissue. Cancer is the second-leading cause of death in the world. Numerous therapies have been developed to treat the various cancer diseases. However, sometimes, cancer cells can overcome the efficacy of the anticancer therapies. It is thus important to specifically investigate and target the mechanism of cancer and offer new and more effective therapies.

As it is known in the art, metabolic pathways leading to the synthesis, uptake and usage of the nonessential amino acid serine are frequently amplified in cancer. Many cancer types display enhanced serine biosynthesis and import of serine from the extracellular medium, as evidenced by the amplified expression and use of the de novo serine synthesis enzymes phosphoglycerate dehydrogenase (PHGDH), phosphoserine aminotransferase (PSAT1), and phosphoserine phosphatase (PSPH) and of the solute carrier family 1 member 4 and 5 (*i.e.* SLC1A4 and SLC1A5). Much of this serine is shuttled to the mitochondria for catabolism to glycine and 1C units. A meta-analysis study revealed that enzymes involved in the mitochondrial 1C pathway are among the most frequently overexpressed genes in cancer (Nilsson et al., 2014, Nat. Commun., Vol. 5 : 3128).

A number of scientific groups have reported on the efficacy and mechanisms of pharmacological inhibitors against Serine Glycine One-Carbon (SGOC). It may notably cited the following pharmacologic inhibitors : (i) SHINI targeting SHMT1/2 (Ducker et al., 2017, Proc Natl Acad Sci USA, Vol. 114 : 11404:11409), (ii) AGF347 targeting SHMT1/2, GART and ATIC (Dekhme et al., 2019, Mol Cancer Ther, Vol. 18 : 1787-1799), (iii) 2.12 targeting SHMT1/2 (Marani et al., 2016, Oncotarget, Vol. 7 : 4570-4583), (iv) LY345899 targeting MTHFD1/2 (Gustafsson et al., 2017, Cancer Res, Vol. 77 : 937-948), (v) Carolacton targeting MTHFD1/2 (Fu et al., 2017, Nat Commun., Vol. 8 : 1529), (vi) LY231514/MTA/Pemetrexed targeting TYMS, DHFR, GART and ATIC (Chattopadhyay et al., 2007, Mol Cancer Ther, Vol. 6 : 404-417), (vii) Amethopterin/MTX/Methothrexate targeting TYMS and DHFR (Kremer, 2004, Artthritis Rheum, Vol. 50 : 1370-1382) and (viii) 5-FU targeting TYMS (Danenberg, 1977, Biochim Biophys Acta, Vol. 473 : 73-92).

In liposarcomas, it was reported an enhanced recruitment of MDM2 to chromatin as well as a high dependency of these cancers on serine and glycine metabolism. In this context, it was reported that genetic or pharmacological targeting of chromatin-bound MDM2, or of the 3-phosphoglycerate dehydrogenase (PHGDH) enzyme that catalyzes the first limiting step of de novo serine synthesis would represent very efficient therapeutic strategies for liposarcomas (PCT application n° WO 2019/106126). In this document, targeting of key genes involved in serine metabolism or transport including PGHDH, PSAT1, PSPH, SLC1A4 and therapeutic strategies limiting serine availability or intake were suggested for treating liposarcomas with chromatin-bound MDM2. Antibodies were cited among the various MDM2 inhibitors that were contemplated, although no antibody was actually prepared nor, by definition, tested.

There remains a need to set up further therapeutic strategies aimed at preventing or treating cancers involving a deregulation of the synthesis, uptake and usage of the nonessential amino acid serine, including an amplification thereof.

There also remains a need to provide for methods allowing determining cancers involving a deregulation of the synthesis, uptake and usage of the nonessential amino acid serine, including an amplification thereof.

The present disclosure has for purpose to satisfy all or part of those needs.

### SUMMARY OF THE DISCLOSURE

The present disclosure relates to a monoclonal antibody directed against the extracellular loop of human SLC1A4 protein, wherein the said monoclonal antibody is selected from :
**a)** a monoclonal antibody, or an antigen-binding fragment thereof, comprising :
   **(i)** a heavy chain wherein the variable domain comprises :
      - a VH-CDR1 having a sequence set forth as SEQ ID NO. 1;
      - a VH-CDR2 having a sequence set forth as SEQ ID NO. 2; and
      - a VH-CDR3 having a sequence set forth as SEQ ID NO. 3;
      and/or
   **(ii)** a light chain wherein the variable domain comprises :
      - a VL-CDR1 having a sequence set forth as SEQ ID NO. 5;
      - a VL-CDR2 having a sequence set forth as SEQ ID NO. 6; and
      - a VL-CDR3 having a sequence set forth as SEQ ID NO. 7.
**b)** a monoclonal antibody, or an antigen-binding fragment thereof, comprising :
   **(i)** a heavy chain wherein the variable domain comprises :
      - a VH-CDR1 having a sequence set forth as SEQ ID NO. 9;
      - a VH-CDR2 having a sequence set forth as SEQ ID NO. 10; and
      - a VH-CDR9 having a sequence set forth as SEQ ID NO. 11;
      and/or
   **(ii)** a light chain wherein the variable domain comprises :
      - a VL-CDR1 having a sequence set forth as SEQ ID NO. 13;
      - a VL-CDR2 having a sequence set forth as SEQ ID NO. 14; and
      - a VL-CDR3 having a sequence set forth as SEQ ID NO. 15.
**c)** a monoclonal antibody, or an antigen-binding fragment thereof, comprising :
   **(i)** a heavy chain wherein the variable domain comprises :
      - a VH-CDR1 having a sequence set forth as SEQ ID NO. 17;
      - a VH-CDR2 having a sequence set forth as SEQ ID NO. 18; and
      - a VH-CDR9 having a sequence set forth as SEQ ID NO. 19;
      and/or
   **(ii)** a light chain wherein the variable domain comprises :
      - a VL-CDR1 having a sequence set forth as SEQ ID NO. 21;
      - a VL-CDR2 having a sequence set forth as SEQ ID NO. 22; and
      - a VL-CDR3 having a sequence set forth as SEQ ID NO. 23.
**d)** a monoclonal antibody, or an antigen-binding fragment thereof, comprising :
   **(i)** a heavy chain wherein the variable domain comprises :
      - a VH-CDR1 having a sequence set forth as SEQ ID NO. 25;
      - a VH-CDR2 having a sequence set forth as SEQ ID NO. 26; and
      - a VH-CDR9 having a sequence set forth as SEQ ID NO. 27;
      and/or
   **(ii)** a light chain wherein the variable domain comprises :
      - a VL-CDR1 having a sequence set forth as SEQ ID NO. 29;
      - a VL-CDR2 having a sequence set forth as SEQ ID NO. 30; and
      - a VL-CDR3 having a sequence set forth as SEQ ID NO. 31.
**e)** a monoclonal antibody, or an antigen-binding fragment thereof, comprising :
   **(i)** a heavy chain wherein the variable domain comprises :
      - a VH-CDR1 having a sequence set forth as SEQ ID NO. 33;
      - a VH-CDR2 having a sequence set forth as SEQ ID NO. 34; and
      - a VH-CDR9 having a sequence set forth as SEQ ID NO. 35;
      and/or
   **(ii)** a light chain wherein the variable domain comprises :
      - a VL-CDR1 having a sequence set forth as SEQ ID NO. 37;
      - a VL-CDR2 having a sequence set forth as SEQ ID NO. 38; and
      - a VL-CDR3 having a sequence set forth as SEQ ID NO. 39.
**f)** a monoclonal antibody, or an antigen-binding fragment thereof, comprising :
   **(i)** a heavy chain wherein the variable domain comprises :
      - a VH-CDR1 having a sequence set forth as SEQ ID NO. 41;
      - a VH-CDR2 having a sequence set forth as SEQ ID NO. 42; and
      - a VH-CDR9 having a sequence set forth as SEQ ID NO. 43;
      and/or
   **(ii)** a light chain wherein the variable domain comprises :
      - a VL-CDR1 having a sequence set forth as SEQ ID NO. 45;
      - a VL-CDR2 having a sequence set forth as SEQ ID NO. 46; and
      - a VL-CDR3 having a sequence set forth as SEQ ID NO. 47.

In some embodiments, the said monoclonal antibody comprises :
**(i)** a heavy chain variable domain having at least 85% amino acid identity with the heavy chain variable domain of SEQ ID NO. 4 and comprising a VH-CDR1 of SEQ ID NO. 1, a VH-CDR2 of SEQ ID NO. 2 and a VH-CDR3 of SEQ ID NO. 3;
   and/or
**(ii)** a light chain variable domain having at least 85% amino acid identity with the light chain variable domain of SEQ ID NO. 8 and comprising a VL-CDR1 of SEQ ID NO. 5, a VL-CDR2 of SEQ ID NO. 6 and a VL-CDR3 of SEQ ID NO. 7.

In some embodiments, the said monoclonal antibody comprises :
**(i)** a heavy chain variable domain having at least 85% amino acid identity with the heavy chain variable domain of SEQ ID NO. 12 and comprising a VH-CDR1 of SEQ ID NO. 9, a VH-CDR2 of SEQ ID NO. 10 and a VH-CDR3 of SEQ ID NO. 11;
   and/or
**(ii)** a light chain variable domain having at least 85% amino acid identity with the light chain variable domain of SEQ ID NO. 16 and comprising a VL-CDR1 of SEQ ID NO. 13, a VL-CDR2 of SEQ ID NO. 14 and a VL-CDR3 of SEQ ID NO. 15.

In some embodiments, the said monoclonal antibody comprises :
**(i)** a heavy chain variable domain having at least 85% amino acid identity with the heavy chain variable domain of SEQ ID NO. 20 and comprising a VH-CDR1 of SEQ ID NO. 17, a VH-CDR2 of SEQ ID NO. 18 and a VH-CDR3 of SEQ ID NO. 19;
   and/or
**(ii)** a light chain variable domain having at least 85% amino acid identity with the light chain variable domain of SEQ ID NO. 24 and comprising a VL-CDR1 of SEQ ID NO. 21, a VL-CDR2 of SEQ ID NO. 22 and a VL-CDR3 of SEQ ID NO. 23.

In some embodiments, the said monoclonal antibody comprises :
**(i)** a heavy chain variable domain having at least 85% amino acid identity with the heavy chain variable domain of SEQ ID NO. 28 and comprising a VH-CDR1 of SEQ ID NO. 25, a VH-CDR2 of SEQ ID NO. 26 and a VH-CDR3 of SEQ ID NO. 27;
   and/or
**(ii)** a light chain variable domain having at least 85% amino acid identity with the light chain variable domain of SEQ ID NO. 32 and comprising a VL-CDR1 of SEQ ID NO. 29, a VL-CDR2 of SEQ ID NO. 30 and a VL-CDR3 of SEQ ID NO. 31.

In some embodiments, the said monoclonal antibody comprises :
**(i)** a heavy chain variable domain having at least 85% amino acid identity with the heavy chain variable domain of SEQ ID NO. 36 and comprising a VH-CDR1 of SEQ ID NO. 33, a VH-CDR2 of SEQ ID NO. 34 and a VH-CDR3 of SEQ ID NO. 35;
   and/or
**(ii)** a light chain variable domain having at least 85% amino acid identity with the light chain variable domain of SEQ ID NO. 40 and comprising a VL-CDR1 of SEQ ID NO. 37, a VL-CDR2 of SEQ ID NO. 38 and a VL-CDR3 of SEQ ID NO. 39;

In some embodiments, the said monoclonal antibody comprises :
**(i)** a heavy chain variable domain having at least 85% amino acid identity with the heavy chain variable domain of SEQ ID NO. 44 and comprising a VH-CDR1 of SEQ ID NO. 41, a VH-CDR2 of SEQ ID NO. 42 and a VH-CDR3 of SEQ ID NO. 43;
   and/or
**(ii)** a light chain variable domain having at least 85% amino acid identity with the light chain variable domain of SEQ ID NO. 48 and comprising a VL-CDR1 of SEQ ID NO. 45, a VL-CDR2 of SEQ ID NO. 46 and a VL-CDR3 of SEQ ID NO. 47.

In some embodiments, the said monoclonal antibody is a human antibody.

This disclosure further relates to an antigen-binding fragment of the said antibody, which can be selected from the group consisting of fv, Fab, F(ab')2, Fab', dsFv, scFv, sc(Fv)2 and diabodies.

In some embodiments, the monoclonal antibody or an antigen-binding fragment thereof can be linked to a therapeutic or diagnostic agent, so as to form an immunoconjugate.

This disclosure also pertains to a nucleic acid sequence encoding a monoclonal antibody described herein or an antigen-binding fragment thereof, as well as to a vector comprising the said nucleic acid and also to a host cell, in particular a procaryotic or eucaryotic host cell, comprising a nucleic acid sequence or a vector as described herein.

This disclosure further concerns the *in vitro* use of a monoclonal antibody as described herein or of an antigen-binding fragment thereof for detecting SLC1A4 in a sample, preferably in a cancer tissue sample.

This disclosure further concerns the *in vivo* use of a monoclonal antibody as described herein or of an antigen-binding fragment thereof or an immunoconjugate for detecting SLC1A4 expressing tumors, e.g. by an immuno-imaging technique, in cancerous patients.

This disclosure also relates to a pharmaceutical composition comprising an antibody as described herein or an antigen-binding fragment thereof or an immunoconjugate as described herein, in combination with a pharmaceutically acceptable carrier.

This disclosure also pertains to a monoclonal antibody as described herein, or an antigen-binding fragment thereof, or an immunoconjugate thereof, for use as a drug.

It also concerns a monoclonal antibody as described herein, or an antigen-binding fragment thereof, or an immunoconjugate thereof, for use for treating a SLC1A4-expressing cancer.

### DESCRIPTION OF THE FIGURES

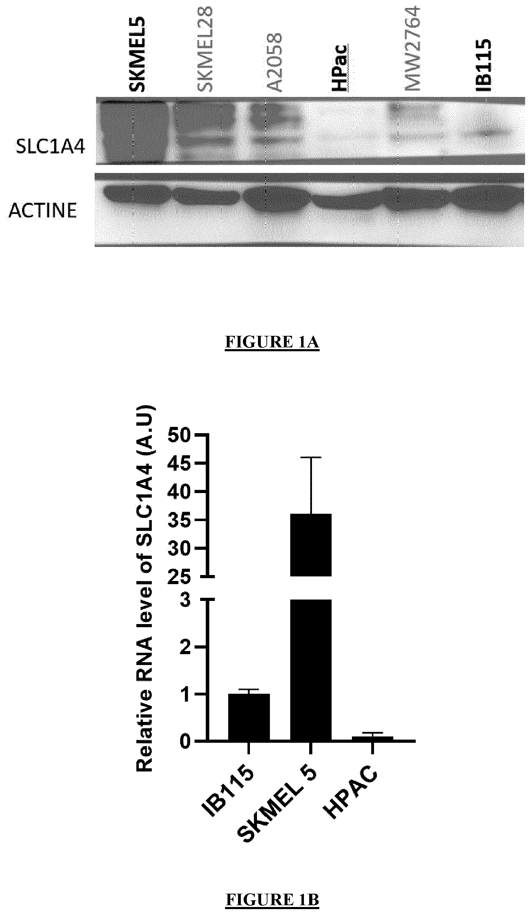

**Figure 1** shows SLC1A4 protein expression by a plurality of human cell lines.
Figure 1A is a photograph from a Western blot assay. Upper line : detection with commercial anti-SLC1A4 antibodies. Bottom line ; detection with commercial anti-beta-actine antibodies. Lanes, from left to right : (i) SK-MEL-5 melanoma cell line; (ii) SK-MEL-28 melanoma cell line; (iii) HPAC pancreatic cancer cell line; (iv) A2058 melanoma cell line; (v) MW2664 melanoma cell line; (vi) IB115 sarcoma cell line.
Figure 1B is a graph illustrating the SLC1A4 mRNA expression level of the SLC1A4 gene in a plurality of cell lines. From left to right : (i) IB115 sarcoma cell line; SK-MEL-5 melanoma cell line; (ii) HPAC pancreatic cancer cell line. Ordinate : SLC1A4 mRNA expression level, as expressed in arbitrary units (UA). Cell lines were selected after analysis of gene expression in the CCLE (Cancer Cell Line Encyclopedia).

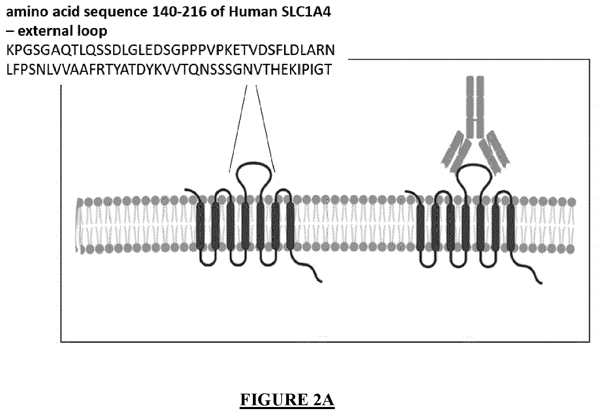

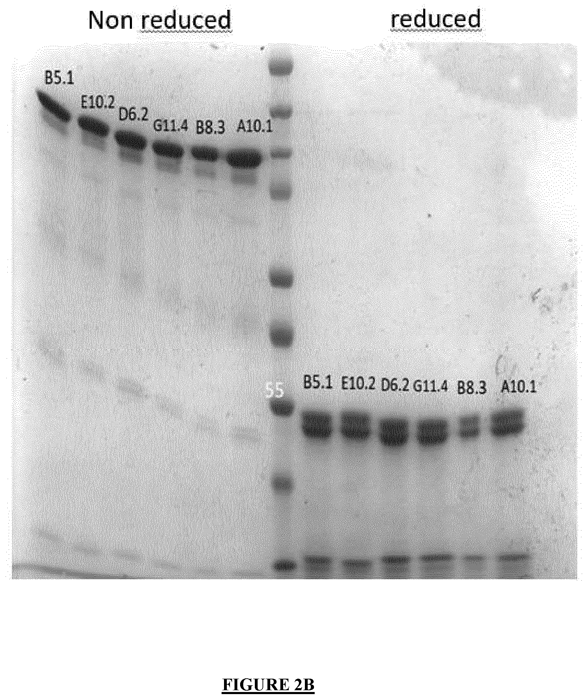

**Figure 2** illustrates the binding of the anti-SLC1A4 monoclonal antibodies of this disclosure to the human SLC1A4 target extracellular loop.
Figure 2A : upper box : amino acid sequence (SEQ ID NO. 51) of the human SLC1A4 extracellular loop. Main box : a scheme of the membrane-embedded SLC1A4 protein including the intra-, extra- and trans-membrane domain, as well as the extracellular loop. Left part : SLC1A4. Right part : illustration scheme of the binding of an anti-SLC1A4 monoclonal antibody to the SLC1A4 extracellular loop.
Figure 2B is a photograph from a Western blot assay of six anti-SLC1A4 human monoclonal antibodies of this disclosure, respectively lanes from left to right : (i) B5.1, (ii) E10.2, (iii) D6.2, (iv) G1.4, (v) B8.3, (vii) A10.1. Left panel : Non-reducing conditions; Right panel : reducing conditions. The human antibodies used in this assay were engineered and their Fc fragment replaced by a rabbit Fc fragment to facilitate their detection by a labelled anti-rabbit IgG commercial antibody. It is known that such an engineering does not modify the specificity of the engineered antibodies, which resides in the variable VH and VL fragments.

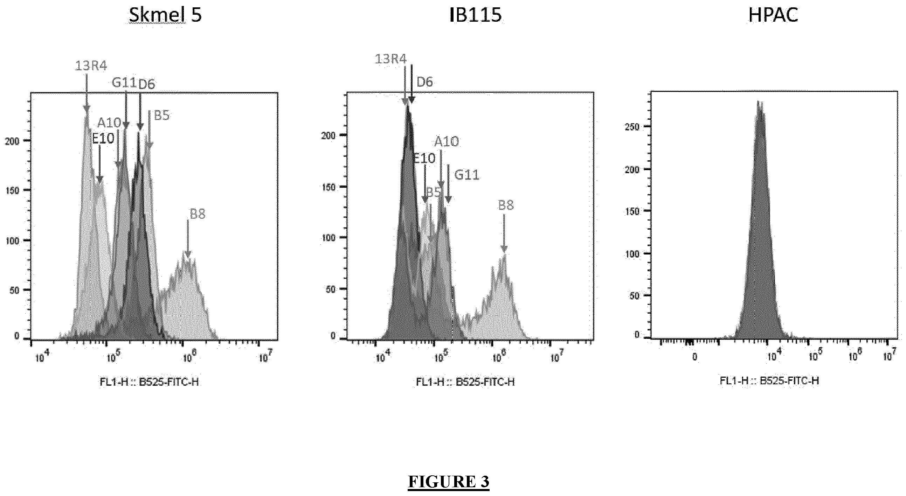

**Figure 3** Illustrates the binding of a plurality of human anti-SLC1A4 monoclonal antibodies of this disclosure to the target SLC1A4 in a flow cytometry assay. Abscissa : fluorescence intensity expressed in arbitrary units. Ordinate : cumulative number of fluorescence-positive cellular events detected by the instrument. Boxed graphs, from left to right : (i) SK-MEL-5 melanoma cell line; (ii) IB115 sarcoma cell line; (iii) HPAC pancreatic cancer cell line. Fluorescence signal peaks labeled for the following antibodies: (i) 13R4 control anti-beta galactosidase antibody, (ii) E10 anti-SLC1A4, (iii) A10 anti-SLC1A4, (iv) G11 anti-SLC1A4, (v) D6 anti-SLC1A4, (vi) B5 anti-SLC1A4 and (vii) B8 anti-SLC1A4. The human antibodies used in this assay were engineered and their Fc fragment replaced by a rabbit Fc fragment to facilitate their detection by a labelled anti-rabbit IgG commercial antibody as well.

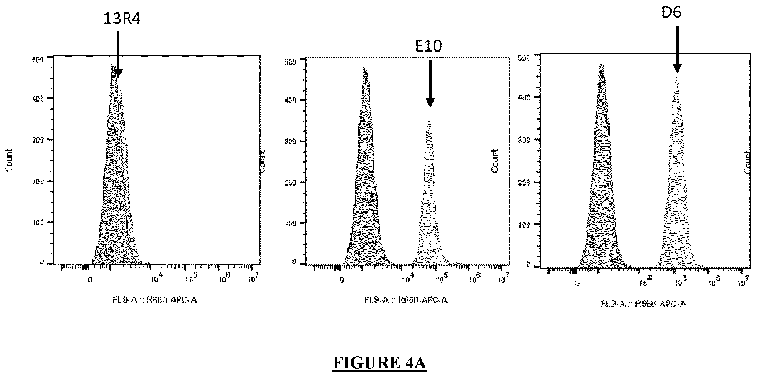

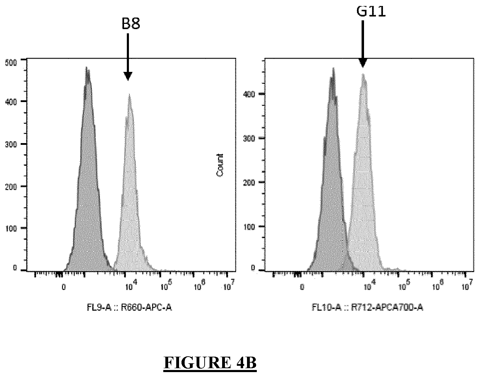

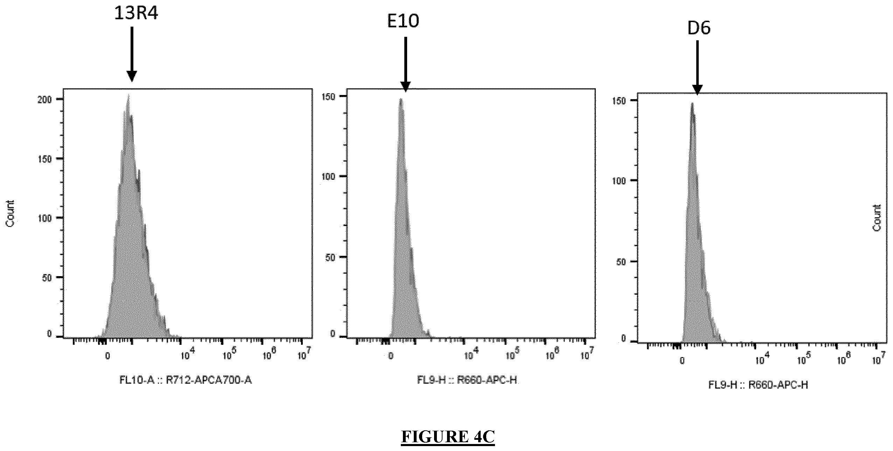

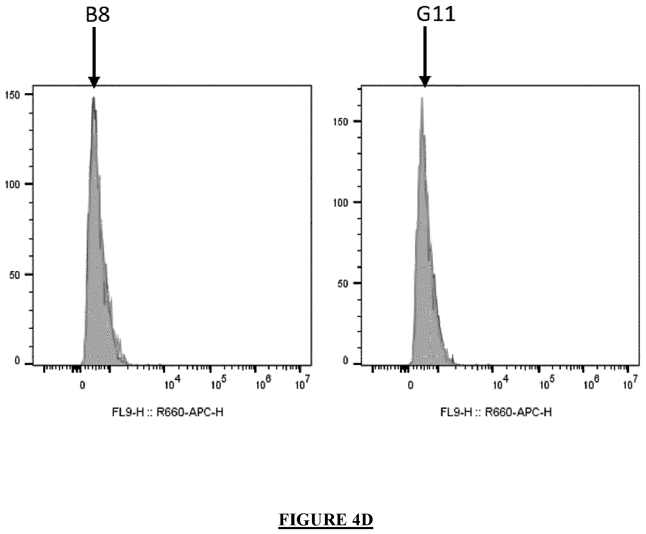

**Figure 4** illustrates the binding of a plurality of human IgG1 monoclonal antibodies to SLC1A4 expressed at the membrane of (i) SK-MEL-5 melanoma cell line (Figures 4A, 4B) or of (ii) HPAC pancreatic cell line (Figures 4C, 4D). Abscissa : fluorescence intensity expressed in arbitrary units. Ordinate : cumulative number of fluorescence-positive cellular events detected by the instrument. Figure 4A, from left to right : (i) 13R4 control anti-beta galactosidase antibody, (ii) E10 anti-SLC1A4, (iii) D6 anti-SLC1A4.
Figure 4B, from left to right: (i) B8 anti-SLC1A4, (ii) G11 anti-SLC1A4.
Figure 4C, from left to right: (i) 13R4 control anti-beta galactosidase antibody, (ii) E10 anti-SLC1A4, (iii) D6 anti-SLC1A4.
Figure 4D, from left to right: (i) B8 anti-SLC1A4, (ii) G11 anti-SLC1A4.

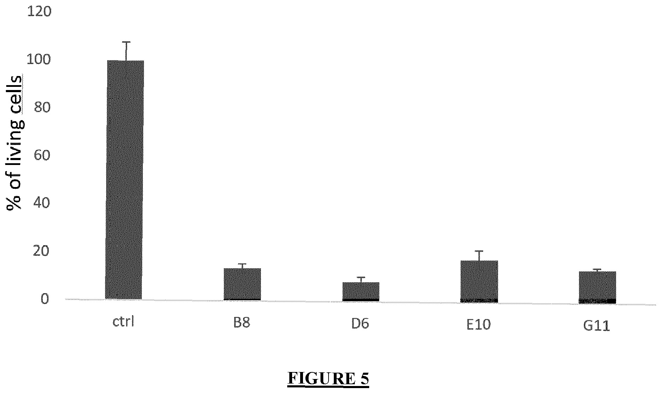

**Figure 5** Illustrates the *in vitro* effect of a plurality of human IgG1 monoclonal anti-SLC 1 A4 antibodies of this disclosure on IB115 liposarcoma cell line. Abscissa, from left to right : (i) control culture in the absence of antibody; (ii) B8 anti-SLC1A4 antibody; (iii) D6 anti-SLC1A4 antibody; (iv) E10 anti-SLC1A4 antibody; (v) G11 anti-SLC1A4 antibody. Ordinate: living cells (%) as compared to the reference (untreated) control cells.

### DETAILED DESCRIPTION

The inventors were aimed at conceiving agents which bind to SLC1A4, especially which bind to SLC1A4-expressing cells, with the purpose of targeting SLC1A4-expressing tumor cells and of killing these tumor cells. More precisely, the inventors were aimed at conceiving novel anti-SLC1A4 monoclonal antibodies that can be used for detecting or for killing SLC1A4-expressing tumor cells.

In some embodiments, these SLC1A4 monoclonal antibodies are aimed at exerting a cytotoxic anti-tumor effect via the blocking of serine transport by SLC1A4 and/or the activation of a an antibody-dependent cellular cytotoxicity natural process (ADCC) and/or, after conjugation to cytotoxic drugs (to form Antibody Drug Conjugates, ADC), these SLC1AA4 monoclonal antibodies could be used as shuttles to make possible the specific entry into cancer cells of cytotoxic drugs which will kill the cancer cells. The inventors' goal was to conceive *in vitro* and/or *in vivo* (*e.g.* by immuno-imaging techniques) diagnostic and therapeutic tools useful in the field of preventing and/or treating SLC1A4-expressing cancers, which are serine-dependent cancers. As used herein, treating SLC1A4-expressing cancers encompasses preventing the development of SLC1A4-expressing cancers, which includes preventing the development of SLC1A4-expressing cancers in patients already treated with other anti-cancer agents, which encompasses patients for which a SLC1A4-expressing cancer has become resistant to one or more of other anti-cancer agents.

To this aim, the inventors have conceived a family of monoclonal antibodies directed against the external loop of the SLC1A4 protein, and more particularly against the external loop of the human SLC1A4 protein. These monoclonal antibodies are also termed "anti-xlSLC1A4" monoclonal antibodies in the present description ("xl" being used to mean "external loop").

The antibodies disclosed herein, because they consist of monoclonal antibodies, which includes human monoclonal antibodies, are directed against a determinable target polypeptide sequence of interest included in the human SLC1A4 polypeptide sequence, in contrast to polyclonal antibodies that comprise antibodies directed against numerous distinct antigenic peptides included in the target protein. Further, the antibodies disclosed herein, because they consist of structurally defined monoclonal antibodies, which includes human monoclonal antibodies, are easily reproducible by a plurality of well known techniques, in contrast to polyclonal antibodies.

The monoclonal antibodies disclosed herein, because they bind to the external loop of SLC1A4 which is exposed at the cellular membrane and is thus easily available for binding. The binding of the monoclonal antibodies disclosed herein at the cell membrane of SLC1A4-expressing tumor cells allow (i) when desired, inducing the killing of the tumor cells through ADCC (antibody-dependent cell cytotoxicity), (ii) when desired, inducing the killing of the tumor cells through CDC (complement-dependent cell cytotoxicity) and (iii) when desired, and when associated with a cytotoxic moiety (typically under and ADC format), inducing the killing of tumor cells by internalization of the monoclonal antibodies associated with the said cytotoxic moiety.

Because the monoclonal antibodies disclosed herein bind to the extracellular loop of the SLC1A4 protein, these monoclonal antibodies may block SLC1A4 for serine transport, which may contribute to the killing of the SLC1A4-expressing tumor cells.

According to the inventors' knowledge, monoclonal antibodies directed against the external loop of human SLC1A4 are disclosed for the first time in the present disclosure.

Monoclonal antibodies directed against the external loop of human SLC1A4 may also be termed "anti-xlSLC1A4" herein.

The inventors have shown that the anti-xlSLC1A4 monoclonal antibodies disclosed herein are able to specifically bind to SLC1A4-expressing cancer cells. Consequently, the anti-xlSLC1A4 monoclonal antibodies disclosed herein can advantageously be used for detecting SLC1A4-expressing cancer cells, for diagnosis purpose.

The inventors have also shown that the anti-xlSLC1A4 monoclonal antibodies disclosed herein have anti-proliferative effects towards SLC1A4-expressing human cancer cells.

Importantly, the inventors have also shown that the *in vitro* anti-proliferative effects are transcribed *in vivo* by the anti-tumour properties of the anti-xlSLC1A4 monoclonal antibodies disclosed herein.

Consequently, it is shown herein that the anti-xlSLC1A4 monoclonal antibodies of the present disclosure are useful as novel therapeutic agents for preventing the development of cancers and/or treating cancers, either alone or as combined with one or more other anti-cancer therapies, especially for preventing and/or treating SLC1A4-expressing cancers, which consist of serine-dependent cancers.

### Definitions

The term "SLC1A4" has its general meaning in the art and refers to Solute Carrier Family 1 Member 4, the neutral amino acid transporter of serine having the UniProtKB accession number P43007. The term "SLC1A4" may be interchangeably used with the term "ASCT1", the Alanine/Serine/Cysteine/Threonine Transporter 1.

As used herein, "xlSLC1A4" means the extracellular loop of the SLC1A4 protein.

As used herein, the external loop of the human SLC1A4 protein, which may also be termed human "xlSLC1A4" herein, consists of the amino acid sequence of SEQ ID NO. 51.

According to the present disclosure, "antibody" or "immunoglobulin" have the same meaning and will be used interchangeably. The term "antibody" as used herein refers to immunoglobulin molecules and immunologically active portions of immunoglobulin molecules, *i.e.,* molecules that contain an antigen binding site that specifically binds an antigen. As such, the term antibody encompasses not only whole antibody molecules, but also variants (including derivatives) of antibodies. In natural antibodies, two heavy chains are linked to each other by disulfide bonds and each heavy chain is linked to a light chain by a disulfide bond. The light and heavy chains of an immunoglobulin each have three CDRs, designated VL-CDR1, VL-CDR2, VL-CDR3 and VH-CDR1, VH-CDR2, VH-CDR3, respectively. An antigen-binding site, therefore, includes at least the three CDRs comprised either within the heavy chain or within the light chain. An antigen-binding site may comprise the six CDRs, comprising the CDR set from each of a heavy and a light chain V region. Framework Regions (FRs) refer to amino acid sequences interposed between CDRs.

In the present text, the terms "antibody" or "immunoglobulin" is used in the broadest sense and includes fully assembled antibodies, monoclonal antibodies, polyclonal antibodies, multispecific antibodies (e.g., bispecific antibodies), antibody fragments that can bind human SLC1A4 external loop, and recombinant peptides comprising the foregoing as long as they exhibit the desired biological activity defined herein.

The terms "monoclonal antibody" or "monoclonal antibody composition" as used herein refer to a preparation of antibody molecules of single molecular composition. A monoclonal antibody composition displays a single binding specificity and affinity for a particular epitope of a target protein, which encompasses the external loop of human SLC1A4.

As used herein the term "human antibody" is intended to include antibodies having variable and constant regions derived from human immunoglobulin sequences repertoire. The human antibodies of the present disclosure may include amino acid residues not directly encoded by the human immunoglobulin sequences repertoire (e.g., mutations introduced by random or site-specific mutagenesis *in vitro* or by somatic mutation *in vivo*).

The term "antigen binding fragment" of an antibody, as used herein, refers to one or more fragments of an intact antibody that retain the ability to specifically binds to a given antigen (*e.g.*, human SLC1A4 extracellular loop). Antigen-binding fragments encompass a Fv, Fab, a F(ab)'2, a single domain antibody, a dsFv, a ScFv, a Sc(Fv)2 and a diabody. Typically, an antigen binding fragment comprises at least (i) the set of VH-CDR1, VH-CDR2 and VH-CDR3 or (ii) the set of VL-CDR1, VL-CDR2 and VL-CDR3, or both of these sets, from a parent antibody.

The term "treatment" or "therapy" refers to administering an active agent with the purpose to cure, heal, alleviate, relieve, alter, remedy, ameliorate, improve, or affect a condition (e.g., a disease), the symptoms of the condition, or to prevent or delay the onset of the symptoms, complications, biochemical indicia of a disease, or otherwise arrest or inhibit further development of the disease, condition, or disorder in a statistically significant manner.

The term "therapeutically effective amount" refers to an amount of a drug effective to treat a disease or disorder in a mammal. In the case of cancer, the therapeutically effective amount of the drug may reduce the number of cancer cells; reduce the tumor size; inhibit (*i.e.,* slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the disorder. To the extent the drug may prevent growth and/or kill existing cancer cells, it may be cytostatic and/or cytotoxic. For cancer therapy, efficacy in vivo can, for example, be measured by assessing the duration of survival, duration of progression free survival (PFS), the response rates (RR), duration of response, and/or quality of life. In the present context, this term encompasses an amount of anti-xlSLC1A4 monoclonal antibody having an anti-tumor effect when administered to a subject affected with a SLC1A4-expressing cancer.

According to the disclosure, the terms "subject", "patient", "individual", "subject in need thereof", "patient in need thereof' or "individual in need thereof' are equivalent and are intended for a human or non-human mammal affected or likely to be affected with cancer associated with the expression of human SLC1A4, also termed a SLC1A4-expressing cancer herein. Said individual is preferably a human being.

As used herein, a "SLC lA4-expressing" cancer is a cancer wherein the tumor cells comprised therein, or derived thereof, express the SLC1A4 protein at their surface. SLC1A4-expressing cells can be identified by binding an anti-xlSLC1A4 monoclonal antibody disclosed herein.

As used herein, a "serine-dependent" cancer is a cancer that is dependent on the availability of serine in its environment for its viability and growth. A serine-dependent cancer is a cancer comprising cancer cells having their growth or proliferation reduced or blocked in the absence of serine.

For each of the amino acid sequences of interest, especially for each of the antibody amino acid sequences of interest, reference sequences are described herein. The present description also encompasses amino acid sequences having specific percentages of amino acid identity with a reference amino acid sequence.

As used herein, the "percentage of identity" between two amino acid sequences, or between two nucleic acid sequences, is determined by comparing both optimally aligned sequences through a comparison window.

The portion of the amino-acid sequence, or nucleic acid sequence, in the comparison window may thus include additions or deletions (for example "gaps") as compared to the reference sequence (which does not include these additions or these deletions) so as to obtain an optimal alignment between both sequences.

The terms "sequence identity" or "identity" are used interchangeably herein. For the purpose of the disclosure, it is defined here that in order to determine the percentage of sequence identity of two amino acid sequences, or of two nucleic acid sequences, the sequences are aligned for optimal comparison purposes. In order to optimize the alignment between the two sequences gaps may be introduced in any of the two sequences that are compared. Such alignment can be carried out over the full length of the sequences being compared. Alternatively, the alignment may be carried out over a shorter length, for example over about 20, about 50, about 100 or more amino acids, or nucleotides. The sequence identity is the percentage of identical matches between the two sequences over the reported aligned region. A comparison of sequences and determination of percentage of sequence identity between two sequences can be accomplished using a mathematical algorithm. The skilled person will be aware of the fact that several different computer programs are available to align two sequences and determine the identity between two sequences (Kruskal, J. B. (1983) An overview of sequence comparison In D. Sankoff and J. B. Kruskal, (ed.), Time warps, string edits and macromolecules: the theory and practice of sequence comparison, pp. 1-44 Addison Wesley).

The percent sequence identity between two amino acid sequences, or between two nucleic acid sequences, is most preferably determined using the Needleman and Wunsch algorithm for the alignment of two sequences. (Needleman, S. B. and Wunsch, C. D. (1970) J. Mol. Biol. 48, 443-453). Both amino acid sequences, or nucleotide sequences, can be aligned by the algorithm. The Needleman-Wunsch algorithm has been implemented in the computer program NEEDLE. The NEEDLE program from the EMBOSS package can be used (version 2.8.0 or higher, EMBOSS: The European Molecular Biology Open Software Suite (2000) Rice, P. LongdenJ. and Bleasby,A. Trends in Genetics 16, (6) pp276- 277, http://emboss.bioinformatics.nl/). For protein sequences EBLOSUM62 can used for the substitution matrix. For nucleotide sequence, EDNAFULL can used. The optional parameters are preferably a gap opening penalty of 10 and a gap extension penalty of 0.5. No end gap penalty is added. In the Output section, Yes has been indicated in response to the question "Brief identity and similarity" and "SRS pairwise" indicated as Output alignment format. After alignment by the program NEEDLE as described above the percentage of sequence identity between a query sequence and a sequence of the disclosure is calculated as follows: Number of corresponding positions in the alignment showing an identical amino acid , or an identical nucleotide, in both sequences divided by the total length of the alignment after subtraction of the total number of gaps in the alignment. The identity defined as herein can be obtained from NEEDLE by using the NOBRIEF option and is labeled in the output of the program as "longest-identity".

### Anti-xlSLC1A4 monoclonal antibodies

As it has already been specified previously herein, the present disclosure provides for a family of monoclonal antibodies directed against the external loop of the SLC1A4 protein, and more particularly against the external loop of the human SLC1A4 protein ("anti-xlSLC1A4" monoclonal antibodies).

The present disclosure relates to a monoclonal antibody directed against the extracellular loop of human SLC1A4 protein, wherein the said monoclonal antibody is selected from :
**a)** a monoclonal antibody, or an antigen-binding fragment thereof, comprising :
   **(i)** a heavy chain wherein the variable domain comprises :
      - a VH-CDR1 having a sequence set forth as SEQ ID NO. 1;
      - a VH-CDR2 having a sequence set forth as SEQ ID NO. 2; and
      - a VH-CDR9 having a sequence set forth as SEQ ID NO. 3;
      and/or
   **(ii)** a light chain wherein the variable domain comprises :
      - a VL-CDR1 having a sequence set forth as SEQ ID NO. 5;
      - a VL-CDR2 having a sequence set forth as SEQ ID NO. 6; and
      - a VL-CDR3 having a sequence set forth as SEQ ID NO. 7.
**b)** a monoclonal antibody, or an antigen-binding fragment thereof, comprising :
   **(i)** a heavy chain wherein the variable domain comprises :
      - a VH-CDR1 having a sequence set forth as SEQ ID NO. 9;
      - a VH-CDR2 having a sequence set forth as SEQ ID NO. 10; and
      - a VH-CDR9 having a sequence set forth as SEQ ID NO. 11;
      and/or
   **(ii)** a light chain wherein the variable domain comprises :
      - a VL-CDR1 having a sequence set forth as SEQ ID NO. 13;
      - a VL-CDR2 having a sequence set forth as SEQ ID NO. 14; and
      - a VL-CDR3 having a sequence set forth as SEQ ID NO. 15.
**c)** a monoclonal antibody, or an antigen-binding fragment thereof, comprising :
   **(i)** a heavy chain wherein the variable domain comprises :
      - a VH-CDR1 having a sequence set forth as SEQ ID NO. 17;
      - a VH-CDR2 having a sequence set forth as SEQ ID NO. 18; and
      - a VH-CDR9 having a sequence set forth as SEQ ID NO. 19;
      and/or
   **(ii)** a light chain wherein the variable domain comprises :
      - a VL-CDR1 having a sequence set forth as SEQ ID NO. 21;
      - a VL-CDR2 having a sequence set forth as SEQ ID NO. 22; and
      - a VL-CDR3 having a sequence set forth as SEQ ID NO. 23.
**d)** a monoclonal antibody, or an antigen-binding fragment thereof, comprising :
   **(i)** a heavy chain wherein the variable domain comprises :
      - a VH-CDR1 having a sequence set forth as SEQ ID NO. 25;
      - a VH-CDR2 having a sequence set forth as SEQ ID NO. 26; and
      - a VH-CDR9 having a sequence set forth as SEQ ID NO. 27;
      and/or
   **(ii)** a light chain wherein the variable domain comprises :
      - a VL-CDR1 having a sequence set forth as SEQ ID NO. 29;
      - a VL-CDR2 having a sequence set forth as SEQ ID NO. 30; and
      - a VL-CDR3 having a sequence set forth as SEQ ID NO. 31.
**e)** a monoclonal antibody, or an antigen-binding fragment thereof, comprising :
   **(i)** a heavy chain wherein the variable domain comprises :
      - a VH-CDR1 having a sequence set forth as SEQ ID NO. 33;
      - a VH-CDR2 having a sequence set forth as SEQ ID NO. 34; and
      - a VH-CDR9 having a sequence set forth as SEQ ID NO. 35;
      and/or
   **(ii)** a light chain wherein the variable domain comprises :
      - a VL-CDR1 having a sequence set forth as SEQ ID NO. 37;
      - a VL-CDR2 having a sequence set forth as SEQ ID NO. 38; and
      - a VL-CDR3 having a sequence set forth as SEQ ID NO. 39.
**f)** a monoclonal antibody, or an antigen-binding fragment thereof, comprising :
   **(i)** a heavy chain wherein the variable domain comprises :
      - a VH-CDR1 having a sequence set forth as SEQ ID NO. 41;
      - a VH-CDR2 having a sequence set forth as SEQ ID NO. 42; and
      - a VH-CDR9 having a sequence set forth as SEQ ID NO. 43;
      and/or
   **(ii)** a light chain wherein the variable domain comprises :
      - a VL-CDR1 having a sequence set forth as SEQ ID NO. 45;
      - a VL-CDR2 having a sequence set forth as SEQ ID NO. 46; and
      - a VL-CDR3 having a sequence set forth as SEQ ID NO. 47.

In some embodiments, a monoclonal antibody directed against the extracellular loop of human SLC1A4 protein according to the present disclosure comprises either the specified CDRs of the heavy chain or the specified CDRs of the light chain.

In some other embodiments, a monoclonal antibody directed against the extracellular loop of human SLC1A4 protein according to the present disclosure comprises both the specified CDRs of the heavy chain and the specified CDRs of the light chain.

The family of monoclonal antibodies described herein encompasses the anti-xlSLC1A4 monoclonal antibodies comprising :
**(i)** a heavy chain variable domain having at least 85% amino acid identity with the heavy chain variable domain of SEQ ID NO. 4 and comprising a VH-CDR1 of SEQ ID NO. 1, a VH-CDR2 of SEQ ID NO. 2 and a VH-CDR3 of SEQ ID NO. 3;
   and/or
**(ii)** a light chain variable domain having at least 85% amino acid identity with the light chain variable domain of SEQ ID NO. 8 and comprising a VL-CDR1 of SEQ ID NO. 5, a VL-CDR2 of SEQ ID NO. 6 and a VL-CDR3 of SEQ ID NO. 7.

The family of monoclonal antibodies described herein encompasses the anti-xlSLC1A4 monoclonal antibodies comprising :
**(i)** a heavy chain variable domain having at least 85% amino acid identity with the heavy chain variable domain of SEQ ID NO. 12 and comprising a VH-CDR1 of SEQ ID NO. 9, a VH-CDR2 of SEQ ID NO. 10 and a VH-CDR3 of SEQ ID NO. 11;
   and/or
**(ii)** a light chain variable domain having at least 85% amino acid identity with the light chain variable domain of SEQ ID NO. 16 and comprising a VL-CDR1 of SEQ ID NO. 13, a VL-CDR2 of SEQ ID NO. 14 and a VL-CDR3 of SEQ ID NO. 15.

The family of monoclonal antibodies described herein encompasses the anti-xlSLC1A4 monoclonal antibodies comprising :
**(i)** a heavy chain variable domain having at least 85% amino acid identity with the heavy chain variable domain of SEQ ID NO. 20 and comprising a VH-CDR1 of SEQ ID NO. 17, a VH-CDR2 of SEQ ID NO. 18 and a VH-CDR3 of SEQ ID NO. 19;
   and/or
**(ii)** a light chain variable domain having at least 85% amino acid identity with the light chain variable domain of SEQ ID NO. 24 and comprising a VL-CDR1 of SEQ ID NO. 21, a VL-CDR2 of SEQ ID NO. 22 and a VL-CDR3 of SEQ ID NO. 23.

The family of monoclonal antibodies described herein encompasses the anti-xlSLC1A4 monoclonal antibodies comprising :
**(i)** a heavy chain variable domain having at least 85% amino acid identity with the heavy chain variable domain of SEQ ID NO. 28 and comprising a VH-CDR1 of SEQ ID NO. 25, a VH-CDR2 of SEQ ID NO. 26 and a VH-CDR3 of SEQ ID NO. 27;
   and/or
**(ii)** a light chain variable domain having at least 85% amino acid identity with the light chain variable domain of SEQ ID NO. 32 and comprising a VL-CDR1 of SEQ ID NO. 29, a VL-CDR2 of SEQ ID NO. 30 and a VL-CDR3 of SEQ ID NO. 31.

The family of monoclonal antibodies described herein encompasses the anti-xlSLC1A4 monoclonal antibodies comprising :
**(i)** a heavy chain variable domain having at least 85% amino acid identity with the heavy chain variable domain of SEQ ID NO. 36 and comprising a VH-CDR1 of SEQ ID NO. 33, a VH-CDR2 of SEQ ID NO. 34 and a VH-CDR3 of SEQ ID NO. 35;
   and/or
**(ii)** a light chain variable domain having at least 85% amino acid identity with the light chain variable domain of SEQ ID NO. 40 and comprising a VL-CDR1 of SEQ ID NO. 37, a VL-CDR2 of SEQ ID NO. 38 and a VL-CDR3 of SEQ ID NO. 39.

The family of monoclonal antibodies described herein encompasses the anti-xlSLC1A4 monoclonal antibodies comprising :
**(i)** a heavy chain variable domain having at least 85% amino acid identity with the heavy chain variable domain of SEQ ID NO. 44 and comprising a VH-CDR1 of SEQ ID NO. 41, a VH-CDR2 of SEQ ID NO. 42 and a VH-CDR3 of SEQ ID NO. 43;
   and/or
**(ii)** a light chain variable domain having at least 85% amino acid identity with the light chain variable domain of SEQ ID NO. 48 and comprising a VL-CDR1 of SEQ ID NO. 45, a VL-CDR2 of SEQ ID NO. 46 and a VL-CDR3 of SEQ ID NO. 47.

As used herein, an amino acid sequence having at least 85% amino acid identity with a given heavy chain variable domain has at least 85%, 86%, 87%, 88%, 89%, 90%, 91%, 92%, 93%, 94%, 95%, 96%, 97%, 98% or at least 99% amino acid identity with the said given heavy chain variable domain. It also encompasses an amino acid sequence consisting of the said given heavy chain variable domain, *i.e.* an amino acid sequence having 100% amino acid identity with the said given heavy chain variable domain.

As used herein, an amino acid sequence having at least 85% amino acid identity with a given light chain variable domain has at least 85%, 86%, 87%, 88%, 89%, 90%, 91%, 92%, 93%, 94%, 95%, 96%, 97%, 98% or at least 99% amino acid identity with the said given light chain variable domain. It also encompasses an amino acid sequence consisting of the said given light chain variable domain, *i.e*. an amino acid sequence having 100% amino acid identity with the said given light chain variable domain.

The present disclosure further relates to a monoclonal antibody directed against the extracellular loop of human SLC1A4 protein, wherein the said monoclonal antibody is selected from :
**a)** a monoclonal antibody, which may be termed "B5" herein, comprising :
   (i) a heavy chain variable domain having a heavy chain variable domain of SEQ ID NO. 4; and/or
   (ii) a light chain variable domain having a light chain variable domain of SEQ ID NO. 8;
**b)** a monoclonal antibody, which may be termed "D6" herein, comprising :
   (i) a heavy chain variable domain having a heavy chain variable domain of SEQ ID NO. 12; and/or
   (ii) a light chain variable domain having a light chain variable domain of SEQ ID NO. 16;
**c)** a monoclonal antibody, which may be termed "A10" herein, comprising :
   (i) a heavy chain variable domain having a heavy chain variable domain of SEQ ID NO. 20; and/or
   (ii) a light chain variable domain having a light chain variable domain of SEQ ID NO. 24;
**d)** a monoclonal antibody, which may be termed "E10" herein, comprising :
   (i) a heavy chain variable domain having a heavy chain variable domain of SEQ ID NO. 28; and/or
   (ii) a light chain variable domain having a light chain variable domain of SEQ ID NO. 32;
**e)** a monoclonal antibody, which may be termed "G11" herein, comprising :
   (i) a heavy chain variable domain having a heavy chain variable domain of SEQ ID NO. 36; and/or
   (ii) a light chain variable domain having a light chain variable domain of SEQ ID NO. 40;
**f)** a monoclonal antibody, which may be termed "B8" herein, comprising :
   (i) a heavy chain variable domain having a heavy chain variable domain of SEQ ID NO. 44; and/or
   (ii) a light chain variable domain having a light chain variable domain of SEQ ID NO. 48;

In some embodiments, a monoclonal antibody directed against the extracellular loop of human SLC1A4 protein selected from B5, D6, A10, E10, G11 and B8 described above comprises either the specified heavy chain or the specified light chain.

In some other embodiments, a monoclonal antibody directed against the extracellular loop of human SLC1A4 protein selected from B5, D6, A10, E10, G11 and B8 described above comprises both the specified heavy chain and the specified light chain.

In some embodiments, an anti-xlSLC1A4 monoclonal antibody of the present disclosure can be a chimeric monoclonal antibody, *i.e.* an antibody having human heavy and light chains variable domains and a non-human Fc domain. The non-human Fc domain can be, *e.g.* a mouse Fc domain, a guinea pig Fc domain or a rabbit Fc domain. For instance, a rabbit Fc domain can consist of the Fc domain of SEQ ID NO. 49 disclosed herein, which Fc domain is illustrated in the examples herein.

In some embodiments, an anti-xlSLC1A4 monoclonal antibody of the present disclosure can be a human monoclonal antibody, *i.e.* an antibody having human heavy and light chains variable domains and a human Fc domain. The human Fc domain can be a human IgG Fc domain, such as a human IgG1 Fc domain. For instance, a human Fc domain can consist of the human IgG1 Fc domain of SEQ ID NO. 50 disclosed herein, which human IgG1 Fc domain is illustrated in the examples herein.

The present disclosure also relates to antigen-binding fragments of a monoclonal antibody directed against the extracellular loop of human SLC1A4 protein as described herein.

Most preferably, an antigen-binding fragment of a monoclonal antibody directed against the extracellular loop of human SLC1A4 protein as described herein comprises at least (i) the set of VH-CDR1, VH-CDR2 and VH-CDR3 or (ii) the set of VL-CDR1, VL-CDR2 and VL-CDR3, or both of these sets, from the parent antibody to which the said binding fragment refers. Illustratively, are encompassed herein antigen binding fragments deriving from any of the B5, D6, A10, E10, G11 and B8 monoclonal antibodies.

In some embodiments, an antigen-binding fragment of a monoclonal antibody directed against the extracellular loop of human SLC1A4 protein comprises the variable domain of the heavy chain or the variable domain of the light chain of the said antibody.

In some other embodiments, an antigen-binding fragment of a monoclonal antibody directed against the extracellular loop of human SLC1A4 protein comprises the variable domain of the heavy chain and the variable domain of the light chain of the said antibody.

Antigen-binding fragments of a monoclonal antibody described herein can in particular be selected from the group consisting of Fv, Fab, F(ab')2, Fab', dsFv, scFv, sc(Fv)2 and diabodies. They may be produced by recombinant DNA techniques or by enzymatic or chemical cleavage of intact antibodies, according to methods well known to the one skilled in the art.

"Single-chain Fv" or "scFv" antibody fragments comprise the VH and VL domains of antibody, wherein these domains are present in a single polypeptide chain. Generally, the Fv polypeptide further comprises a polypeptide linker between the VH and VL domains, which enables the scFv to form the desired structure for antigen binding. For a review of scFv, see Pluckthun in The Pharmacology of Monoclonal Antibodies, Vol 113, Rosenburg and Moore eds. Springer- Verlag, New York, pp. 269-315 (1994).

The term "diabodies" refers to small antibody fragments with two antigen-binding sites, which fragments comprise a heavy chain variable domain (VH) connected to a light chain variable domain (VL) in the same polypeptide chain (VH and VL). By using a linker that is too short to allow pairing between the two domains on the same chain, the domains are forced to pair with the complementary domains of another chain and create two antigen- binding sites. Diabodies are described more fully in, for example, EP 404,097; WO 93/11161; and Hollinger et al., Proc. Natl. Acad. Sci. USA, 90:6444-6448 (1993).

Diabodies or bi-specific antibodies can be roughly divided into two categories: immunoglobulin G (IgG)-like molecules and non-IgG-like molecules. IgG-like bsAbs retain Fc-mediated effector functions such as antibody-dependent cell-mediated cytotoxicity (ADCC), complement-dependent cytotoxicity (CDC), and antibody-dependent cellular phagocytosis (ADCP) (Spiess et al., 2015, Mol Immunol., Vol. 67(2) : 95-106.). The Fc region of bsAbs facilitates purification and improves solubility and stability. Bi-specific antobodies in IgG-like formats usually have longer serum half-lives owing to their larger size and FcRn-mediated recycling (Kontermann et al., 2015, Bispecific antibodies. Drug Discov Today Vol. 20(7) : 838-47). Non-IgG-like bsAbs are smaller in size, leading to enhanced tissue penetration (Kontermann et al., 2015, Bispecific antibodies. Drug Discov Today Vol. 20(7): 838-47).

### Methods of producing antibodies of the present disclosure

Anti-xlSLC1A4 monoclonal antibodies of the present disclosure can be produced, after they have been selected, cloned and sequenced as novel antibody entities showing innovative functional properties, by any technique known in the art, such as, without limitation, any chemical, biological, genetic or enzymatic technique, either alone or in combination.

Knowing the amino acid sequence of the desired polypeptide, e.g. the amino acid sequence of the heavy chain or of the light chain of a monoclonal antibody, the one skilled in the art can readily produce any monoclonal antibody disclosed herein, or antigen-binding fragment thereof, by standard techniques for production of polypeptides. For instance, they can be synthesized using well-known solid phase method, preferably using a commercially available peptide synthesis apparatus (such as that made by Applied Biosystems, Foster City, California) and following the manufacturer's instructions. Alternatively, antibodies of the disclosure can be synthesized by recombinant DNA techniques well-known in the art. For example, antibodies can be obtained as DNA expression products after incorporation of DNA sequences encoding the antibodies into expression vectors and introduction of such vectors into suitable eukaryotic or prokaryotic hosts that will express the desired antibodies, from which they can be later isolated using well-known techniques.

### Nucleic acid sequence

Accordingly, a further object of the disclosure relates to a nucleic acid sequence encoding an antibody according to the disclosure. Based on a known amino acid sequence, the one skilled in the art can easily design the appropriate polypeptide-encoding nucleic acid sequence, including by taking into account the preferred codon usage used by the organism (e.g. bacteria, yeast, animal cell, human cell) wherein the antibody synthesis is contemplated.

Typically, said nucleic acid is a DNA or RNA molecule, which may be included in any suitable vector, such as a plasmid, cosmid, episome, artificial chromosome, phage or a viral vector, as it is well known in the art.

### Vectors

The terms "vector", "cloning vector" and "expression vector" mean the vehicle by which a DNA or RNA sequence (e.g. a foreign gene) can be introduced into a host cell, so as to transform the host and promote expression (e.g. transcription and translation) of the introduced sequence.

Another object of the disclosure relates to a vector comprising a nucleic acid of the disclosure.

Any expression vector for animal cell can be used, so long as a gene encoding the human antibody C region can be inserted and expressed. Examples of suitable vectors include pAGE107 (Miyaji H et al. 1990), pAGE103 (Mizukami T et al. 1987), pHSG274 (Brady G et al. 1984), pKCR (O'Hare K et al. 1981), pSG1 beta d2-4-(Miyaji H et al. 1990) and the like.

### Host cells

A further object of the present disclosure relates to a host cell which has been transfected, infected or transformed by a nucleic acid and/or a vector disclosed herein.

The term "transformation" means the introduction of a "foreign" (*i.e.* extrinsic or extracellular) gene, DNA or RNA sequence to a host cell, so that the host cell will express the introduced gene or sequence to produce a desired substance, typically a protein or enzyme coded by the introduced gene or sequence. A host cell that receives and expresses introduced DNA or RNA bas been "transformed".

The nucleic acids of the disclosure can be used to produce an anti-SLC1A4 monoclonal antibody of the present disclosure in a suitable expression system. The term "expression system" means a host cell and compatible vector under suitable conditions, *e.g*. for the expression of a protein coded for by foreign DNA carried by the vector and introduced to the host cell.

Common expression systems include *E. coli* host cells and plasmid vectors, insect host cells and Baculovirus vectors, and mammalian host cells and vectors. Other examples of host cells include, without limitation, prokaryotic cells (such as bacteria) and eukaryotic cells (such as yeast cells, mammalian cells, insect cells, plant cells, etc.). A host according to the disclosure may in particular be a prokaryotic or eukaryotic cell. Specific examples include *E.coli, Kluyveromyces* or *Saccharomyces* yeasts, mammalian cell lines (*e.g.,* Vero cells, CHO cells, 3T3 cells, COS cells, etc.) as well as primary or established mammalian cell cultures *(e.g.,* produced from lymphoblasts, fibroblasts, embryonic cells, epithelial cells, nervous cells, adipocytes, etc.). Examples also include mouse SP2/0-Ag14 cell (ATCC CRL1581), mouse P3X63-Ag8.653 cell (ATCC CRL1580), CHO cell in which a dihydrofolate reductase gene (hereinafter referred to as "DHFR gene") is defective (Urlaub G et al; 1980), rat YB2/3HL.P2.G11.16Ag.20 cell (ATCC CRL1662, hereinafter referred to as "YB2/0 cell"), and the like.

### Immunoconjugates

In the present disclosure, the term "immunoconjugate" is used for defining an anti-xlSLC1A4 that is associated with another moiety, and wherein the said other moiety is linked to the said anti-xlSLC1A4 monoclonal antibody either covalently or non-covalently.

Immunoconjugates of the present disclosure encompass anti-xlSLC1A4 monoclonal antibodies that are linked, most preferably covalently linked, to a detectable molecule, which detectable molecule may also be termed a "detectable label" herein. Such immunoconjugates can be used *in vitro* or *in vivo* for detecting the presence of SLC1A4-expressing tumor cells.

Immunoconjugates of the present disclosure also encompass anti-xlSLC1A4 monoclonal antibodies that are linked, most preferably covalently linked, to a cytotoxic moiety, so that those immunoconjugates can exert a cytotoxic effect by internalization in SLC1A4-expressing tumor cells.

### Detectable label

An antibody of the disclosure can be conjugated with a detectable label to form an anti-xlSLC1A4 immunoconjugate to be used for *in vitro* diagnostic or *in vivo* immuno-imaging of the tumor in cancerous patients. Suitable detectable labels include, for example, a radioisotope, a fluorescent label, a chemiluminescent label, an enzyme label, a bioluminescent label or colloidal gold. Methods of making and detecting such detectably-labeled immunoconjugates are well-known to those of ordinary skill in the art, and are described in more detail below.

In some embodiments, the detectable label is appropriately selected for determining the presence of SLC1A4-expressing cells *in vitro.* For example, the detectable label can be selected among radioactive compounds, fluorescent compounds, chemiluminescent compounds, bioluminescent compounds and enzyme compounds.

In some other embodiments, the detectable label is appropriately selected for determining the presence of SLC1A4-expressing cells *in vivo.* For example, the detectable label can be selected among radioactive compounds, which include positron emitter compounds.

The detectable label can be a radioisotope that is detected by autoradiography. Isotopes that are particularly useful for the purpose of the present disclosure are ³H, ¹²⁵I, ¹³¹I, ³⁵S and ¹⁴C.

Anti-xlSLC1A4 immunoconjugates of the disclosure can also be labeled with a fluorescent compound. The presence of a fluorescently-labeled antibody is determined by exposing the immunoconjugate to light of the proper wavelength and detecting the resultant fluorescence. Fluorescent labeling compounds include fluorescein isothiocyanate, rhodamine, phycoerytherin, phycocyanin, allophycocyanin, o-phthaldehyde and fluorescamine.

Alternatively, anti-xlSLC1A4 immunoconjugates of the disclosure can be detectably labeled by coupling an antibody to a chemiluminescent compound. The presence of the chemiluminescent-tagged immunoconjugate is determined by detecting the presence of luminescence that arises during the course of a chemical reaction. Examples of chemiluminescent labeling compounds include luminol, isoluminol, an aromatic acridinium ester, an imidazole, an acridinium salt and an oxalate ester.

Similarly, a bioluminescent compound can be used to label anti-xlSLC1A4 immunoconjugates of the present disclosure. Bioluminescence is a type of chemiluminescence found in biological systems in which a catalytic protein increases the efficiency of the chemiluminescent reaction. The presence of a bioluminescent protein is determined by detecting the presence of luminescence. Bioluminescent compounds that are useful for labeling include luciferin, luciferase and aequorin.

Alternatively, anti-xlSLC1A4 immunoconjugates can be detectably labeled by linking an anti-human-xlSLC1A4 monoclonal antibody to an enzyme. When the anti-xlSLC1A4-enzyme conjugate is incubated in the presence of the appropriate substrate, the enzyme moiety reacts with the substrate to produce a chemical moiety which can be detected, for example, by spectrophotometric, fluorometric or visual means. Examples of enzymes that can be used to detectably label polyspecific immunoconjugates include β-galactosidase, glucose oxidase, peroxidase and alkaline phosphatase.

Those of skill in the art will know of other suitable labels which can be employed in accordance with the present disclosure. The binding of marker moieties to anti-human-xlSLC1A4 monoclonal antibodies can be accomplished using standard techniques known to the art. Typical methodology in this regard is described by Kennedy et al., Clin. Chim. Acta 70:1, 1976; Schurs et al., Clin. Chim. Acta 81:1, 1977; Shih et al., Int'l J. Cancer 46:1101, 1990; Stein et al., Cancer Res. 50:1330, 1990; and Coligan, *supra.*

Moreover, the convenience and versatility of in vitro immunochemical detection can be enhanced by using anti-human-xlSLC1A4 monoclonal antibodies that have been conjugated with avidin, streptavidin, and biotin. (*See, e.g.,* Wilchek et al. (eds.), "Avidin-Biotin Technology," Methods In Enzymology (Vol. 184) (Academic Press 1990); Bayer et al., "Immunochemical Applications of Avidin-Biotin Technology," in Methods In Molecular Biology (Vol. 10) 149-162 (Manson, ed., The Humana Press, Inc. 1992).)

Methods for performing immunoassays are well-established. (*See, e.g.,* Cook and Self, "Monoclonal Antibodies in Diagnostic Immunoassays," in Monoclonal Antibodies: Production, Engineering, and Clinical Application 180-208 (Ritter and Ladyman, eds., Cambridge University Press 1995); Perry, "The Role of Monoclonal Antibodies in the Advancement of Immunoassay Technology," in Monoclonal Antibodies: Principles and Applications 107-120 (Birch and Lennox, eds., Wiley-Liss, Inc. 1995); Diamandis, Immunoassay (Academic Press, Inc. 1996).

The use of an immunoconjugate according to the present disclosure in an *in vivo* immuno-imaging method encompasses diagnostic imaging through radioimmunoscintigraphy techniques or through positron-emission tomography (PET) techniques. Immuno-positron emission tomography (PET) is a diagnostic imaging tool that utilizes monoclonal antibodies labeled with positron emitters, combining the targeting properties of an antibody with the sensitivity of positron emission tomography cameras (See, *e.g.,* The Oncologist, 12: 1379 (2007); Journal of Nuclear Medicine, 52(8): 1171 (2011). Immuno-PET enables the visualization and quantification of antigen and antibody accumulation *in vivo* and, as such, can serve as an important tool for diagnostics and complementing therapy. For example, immuno - PET can aid in the selection of potential patient candidates for a particular therapy, as well as in the monitoring of treatment. In the context of the present disclosure, the *in vivo* use of immunoconjugates of anti-xlSLC1A4 monoclonal antibodies linked to a detectable label, such as linked to a positron emitter, allows determining whether a subject is affected with a SLC1A4-expressing cancer, allows localizing the SLC1A4-expressing tumor cells within the subject's body, and thus also allows a monitoring of the cancer status withing the subject's body, such as the responsiveness to a selected anti-cancer treatment, which includes responsiveness to an anti-cancer treatment with anti-xlSLC1A4 monoclonal antibodies disclosed herein, and cytotoxic immunoconjugates thereof.

### Antibody-drug conjugates (ADC)

In another aspect, the present disclosure provides an anti-xlSLC1A4 monoclonal antibody-drug conjugate. An "anti-xlSLC1A4 monoclonal antibody-drug conjugate" as used herein refers to an anti-xlSLC1A4 monoclonal antibody according to the present disclosure which is conjugated to a therapeutic agent.

The present disclosure also relates to an immunoconjugate comprising an anti-xlSLC1A4 monoclonal antibody as described herein, or an antigen-binding fragment thereof, linked to a therapeutic agent.

Such an anti-xlSLC1A4 monoclonal antibody-drug conjugate (ADC) is expected to provide clinically beneficial effects in the prevention and/or the treatment of a SLC1A4-expressing cancer, when the said ADC is administered to a subject affected with a SLC1A4-expressing cancer, typically when administered alone but also in combination with one or more other therapeutic agents.

In typical embodiments, an anti-xlSLC1A4 monoclonal antibody of the present disclosure is conjugated to a cytotoxic agent, such that the resulting antibody-drug conjugate (ADC) exerts a cytotoxic or cytostatic effect on a SLC1A4-expressing cell (e.g., a SLC1A4-expressing cancer cell) when taken up or internalized by the cell. Particularly suitable moieties for conjugation to antibodies are chemotherapeutic agents, prodrug converting enzymes, radioactive isotopes or compounds, or toxins. For example, an anti-xlSLC1A4 monoclonal antibody can be conjugated to a cytotoxic agent such as a chemotherapeutic agent or a toxin *(e.g.,* a cytostatic or cytocidal agent such as, for example, abrin, ricin A, pseudomonas exotoxin, or diphtheria toxin).

Useful classes of cytotoxic agents include, for example, antitubulin agents, auristatins, DNA minor groove binders, DNA replication inhibitors, alkylating agents (*e.g.,* platinum complexes such as cis-platin, mono(platinum), bis(platinum) and tri-nuclear platinum complexes and-carboplatin), anthracyclines, antibiotics, antifolates, antimetabolites, chemotherapy sensitizers, duocarmycins, etoposides, fluorinated pyrimidines, ionophores, lexitropsins, nitrosoureas, platinols, pre-forming compounds, purine antimetabolites, puromycins, radiation sensitizers, steroids, taxanes, topoisomerase inhibitors, vinca alkaloids, or the like.

Individual cytotoxic agents include, for example, an androgen, anthramycin (AMC), asparaginase, 5-azacytidine, azathioprine, bleomycin, busulfan, buthionine sulfoximine, camptothecin, carboplatin, carmustine (BSNU), CC-1065 (Li et al., Cancer Res. 42:999-1004, 1982), chlorambucil, cisplatin, colchicine, cyclophosphamide, cytarabine, cytidine arabinoside, cytochalasin B, dacarbazine, dactinomycin (formerly actinomycin), daunorubicin, decarbazine, docetaxel, doxorubicin, an estrogen, 5-fluordeoxyuridine, etopside phosphate (VP-16), 5-fluorouracil, gramicidin D, hydroxyurea, idarubicin, ifosfamide, irinotecan, lomustine (CCNU), mechlorethamine, melphalan, 6-mercaptopurine, methotrexate, mithramycin, mitomycin C, mitoxantrone, nitroimidazole, paclitaxel, plicamycin, procarbizine, streptozotocin, tenoposide (VM-26), 6-thioguanine, thioTEPA, topotecan, vinblastine, vincristine, and vinorelbine.

Particularly suitable cytotoxic agents to be grafted on antibodies to form an ADC include, for example, dolastatins (*e.g*., auristatin E, AFP, MMAF, MMAE), DNA minor groove binders *(e.g.,* enediynes and lexitropsins), duocarmycins, taxanes *(e.g.,* paclitaxel and docetaxel), puromycins, vinca alkaloids, CC-1065, SN-38 (7-ethyl-10-hydroxy-camptothecin), topotecan, morpholino-doxorubicin, rhizoxin, cyanomorpholino-doxorubicin, echinomycin, combretastatin, netropsin, epothilone A and B, estramustine, cryptophysins, cemadotin, maytansinoids, discodermolide, eleutherobin, benzodiazepine derivatives and mitoxantrone.

In certain embodiments, a cytotoxic agent is a conventional chemotherapeutic such as, for example, doxorubicin, paclitaxel, melphalan, vinca alkaloids, methotrexate, mitomycin C or etoposide. In addition, potent agents such as CC-1065 analogues, calicheamicin, maytansine, analogues of dolastatin 10, rhizoxin, and palytoxin can be linked to an anti-xlSLC1A4-expressing antibody.

In other variations, the cytotoxic agent is a DNA minor groove binding agent. (*See, e.g.,* U.S. Patent No. 6,130,237.) For example, in certain embodiments, the minor groove binding agent is a CBI compound. In other embodiments, the minor groove binding agent is an enediyne (*e.g*., calicheamicin).

In other embodiments, an anti-xlSLC1A4 monoclonal antibody of the present disclosure is conjugated to a pro-drug converting enzyme. The pro-drug converting enzyme can be recombinantly fused to the antibody or chemically conjugated thereto using known methods. Exemplary pro-drug converting enzymes are carboxypeptidase G2, β-glucuronidase, penicillin-V-amidase, penicillin-G-amidase, β-lactamase, β-glucosidase, nitroreductase and carboxypeptidase A.

Techniques for conjugating therapeutic agents to proteins, and in particular to antibodies, are well-known. (*See, e.g.,* Arnon et al., "Monoclonal Antibodies For Immunotargeting Of Drugs In Cancer Therapy," in Monoclonal Antibodies And Cancer Therapy (Reisfeld et al. eds., Alan R. Liss, Inc., 1985); Hellstrom et al., "Antibodies For Drug Delivery," in Controlled Drug Delivery (Robinson et al. eds., Marcel Deiker, Inc., 2nd ed. 1987); Thorpe, "Antibody Carriers Of Cytotoxic Agents In Cancer Therapy: A Review," in Monoclonal Antibodies '84: Biological And Clinical Applications (Pinchera et al. eds., 1985); "Analysis, Results, and Future Prospective of the Therapeutic Use of Radiolabeled Antibody In Cancer Therapy," in Monoclonal Antibodies For Cancer Detection And Therapy (Baldwin et al. eds., Academic Press, 1985); and Thorpe et al., 1982, Immunol. Rev. 62:119-58. *See also, e.g.,* PCT publication WO 89/12624.).

### Linkers in Antibody-Drug Conjugates

Typically, the antibody-drug conjugate compounds comprise a linker unit between the drug unit and the antibody unit. In some embodiments, the linker is cleavable under intracellular conditions, such that cleavage of the linker releases the drug unit from the antibody in the intracellular environment. In yet other embodiments, the linker unit is not cleavable and the drug is released, for example, by antibody degradation.

In some embodiments, the linker is cleavable by a cleaving agent that is present in the intracellular environment (e.g., within a lysosome or endosome or caveolea). The linker can be, e.g., a peptidyl linker that is cleaved by an intracellular peptidase or protease enzyme, including, but not limited to, a lysosomal or endosomal protease. In some embodiments, the peptidyl linker is at least two amino acids long or at least three amino acids long. Cleaving agents can include cathepsins B and D and plasmin, all of which are known to hydrolyze dipeptide drug derivatives resulting in the release of active drug inside target cells (see, e.g., Dubowchik and Walker, 1999, Pharm. Therapeutics 83:67-123).

Most typical are peptidyl linkers that are cleavable by enzymes that are present in 191P4D12-expressing cells. Examples of such linkers are described, e.g., in U.S. Pat. No. 6,214,345, incorporated herein by reference in its entirety and for all purposes. In a specific embodiment, the peptidyl linker cleavable by an intracellular protease is a Val-Cit linker or a Phe-Lys linker (see, e.g., U.S. Pat. No. 6,214,345, which describes the synthesis of doxorubicin with the Val-Cit linker). One advantage of using intracellular proteolytic release of the therapeutic agent is that the agent is typically attenuated when conjugated and the serum stabilities of the conjugates are typically high.

In other embodiments, the cleavable linker is pH-sensitive, i.e., sensitive to hydrolysis at certain pH values.

Typically, the pH-sensitive linker hydrolyzable under acidic conditions. For example, an acid-labile linker that is hydrolyzable in the lysosome (e.g., a hydrazone, semicarbazone, thiosemicarbazone, cis-aconitic amide, orthoester, acetal, ketal, or the like) can be used. (See, e.g., U.S. Pat. Nos. 5,122,368; 5,824,805; 5,622,929; Dubowchik and Walker, 1999, Pharm. Therapeutics 83:67-123; Neville et al., 1989, Biol. Chem. 264:14653-14661.) Such linkers are relatively stable under neutral pH conditions, such as those in the blood, but are unstable at below pH 5.5 or 5.0, the approximate pH of the lysosome. In certain embodiments, the hydrolyzable linker is a thioether linker (such as, e.g., a thioether attached to the therapeutic agent via an acylhydrazone bond (see, e.g., U.S. Pat. No. 5,622,929).

In yet other embodiments, the linker is cleavable under reducing conditions (e.g., a disulfide linker). A variety of disulfide linkers are known in the art, including, for example, those that can be formed using SATA (N-succinimidyl-S-acetylthioacetate), SPDP (N-succinimidyl-3-(2-pyridyldithio)propionate), SPDB (N-succinimidyl-3-(2-pyridyldithio)butyrate) and SMPT (N-succinimidyl-oxycarbonyl-alpha-methyl-alpha-(2-pyridyl-dithio)toluene), SPDB and SMPT. (See, e.g., Thorpe et al., 1987, Cancer Res. 47:5924-5931; Wawrzynczak et al., In Immunoconjugates: Antibody Conjugates in Radioimagery and Therapy of Cancer (C. W. Vogel ed., Oxford U. Press, 1987. See also U.S. Pat. No. 4,880,935.)

In yet other embodiments, the linker unit is not cleavable and the drug is released by antibody degradation.

Typically, the linker is not substantially sensitive to the extracellular environment. As used herein, "not substantially sensitive to the extracellular environment," in the context of a linker, means that no more than about 20%, typically no more than about 15%, more typically no more than about 10%, and even more typically no more than about 5%, no more than about 3%, or no more than about 1% of the linkers, in a sample of antibody-drug conjugate compound, are cleaved when the antibody-drug conjugate compound is present in an extracellular environment (e.g., in plasma). Whether a linker is not substantially sensitive to the extracellular environment can be determined, for example, by incubating with plasma the antibody-drug conjugate compound for a predetermined time period (e.g., 2, 4, 8, 16, or 24 hours) and then quantitating the amount of free drug present in the plasma.

### Modified anti-xlSLC1A4 monoclonal antibodies

It may be desirable to modify an anti-xlSLC1A4 antibody specified herein with respect to effector function, *e.g.* so as to enhance antigen-dependent cell-mediated cyotoxicity (ADCC) and/or complement dependent cytotoxicity (CDC) of the antibody. This may be achieved by introducing one or more amino acid substitutions in an Fc region of the antibody. Alternatively or additionally, cysteine residue(s) may be introduced in the Fc region, thereby allowing interchain disulfide bond formation in this region. The homodimeric antibody thus generated may have improved internalization capability and/or increased complement- mediated cell killing and antibody-dependent cellular cytotoxicity (ADCC). See Caron et al, J. Exp Med. 176:1191-1195 (1992) and Shopes, B. J. Immunol. 148:2918-2922 (1992). Homodimeric antibodies with enhanced anti-tumor activity may also be prepared using heterobifunctional cross-linkers as described in Wolff et al. Cancer Research 53:2560-2565 (1993). Alternatively, an antibody can be engineered which has dual Fc regions and may thereby have enhanced complement lysis and ADCC capabilities. See Stevenson et al. Anti- Cancer Drug Design 3:219-230 (1989). WO00/42072 (Presta, L.) describes antibodies with improved ADCC function in the presence of human effector cells, where the antibodies comprise amino acid substitutions in the Fc region thereof. Preferably, the antibody with improved ADCC comprises substitutions at positions 298, 333, and/or 334 of the Fc region (Eu numbering of residues). Preferably the altered Fc region is a human IgGl Fc region comprising or consisting of substitutions at one, two or three of these positions. Such substitutions are optionally combined with substitution(s) which increase CIq binding and/or CDC.

Antibodies with altered CIq binding and/or complement dependent cytotoxicity (CDC) are described in WO99/51642, US Patent No. 6,194,551B1, US Patent No. 6,242,195B1, US Patent No. 6,528,624B1 and US Patent No. 6,538,124 (Idusogie et al). The antibodies comprise an amino acid substitution at one or more of amino acid positions 270, 322, 326, 327, 329, 313, 333 and/or 334 of the Fc region thereof (Eu numbering of residues).

In some embodiments, anti-xlSLC1A4 monoclonal antibodies of the present disclosure encompass glyco-engineered anti-xlSLC1A4 monoclonal antibodies.

As used herein, the term "glycoengineering" refers to any art-recognized method for altering the glycoform profile of a binding protein composition. Such methods include expressing a binding protein composition in a genetically engineered host cell (e.g., a CHO cell) that has been genetically engineered to express a heterologous glycosyltransferase or glycosidase. In other embodiments, the glycoengineering methods comprise culturing a host cell under conditions that bias for particular glycoform profiles.

As used herein, a "glyco-engineered antibody" encompasses (i) an antibody comprising a hyper-galactosylated Fc fragment, (ii) an antibody comprising a hypo mannosylated Fc fragment, which encompasses a amannosylated Fc fragment, and (iii) an antibody comprising a hypo fucosylated Fc fragment, which encompasses a afucosylated Fc fragment. As used herein, a glyco-engineered fragment encompasses a Fc fragment having an altered glycosylation which is selected in a group comprising one or more of the following altered glycosylation (i) hyper-galactosylation, (ii) hypo-mannosylation and (iii) hypo-fucosylation. Consequently, a glyco-engineered Fc fragment from an anti-xlSLC1A4 monoclonal antibody as used according to the disclosure encompass the illustrative examples of a hyper-galactosylated, a hypo-mannosylated and a hypo-fucosylated Fc fragment.

The one skilled in the art may refer to well-known techniques for obtaining anti-xlSLC1A4 monoclonal antibodies comprising hyper-galactosylated Fc fragments, hypo mannosylated Fc fragments and hypo fucosylated Fc fragments that are known to bind to Fc receptors with a higher affinity than non-modified Fc fragments.

Glyco-engineered anti-xlSLC1A4 monoclonal antibodies anti-xlSLC1A4 monoclonal antibodiesd comprising a hypofucosylated Fc fragment, which may also be termed a "low fucose" Fc fragment.

### Antibody assays

As it is shown in the examples herein, the inventors have found that SLC1A4 is expressed at the cell membrane of various cancer tissues, including melanoma and liposarcoma.

As it will be explained in more detail below, the growth of SLC1A4-expressing tumor cells is, at least partly, promoted through the provision of serine which is internalized within the cell by the SLC1A4 neutral amino acid transporter. As it is shown in the examples herein, targeting SLC1A4 with an anti-xlSLC1A4 monoclonal antibody of the present disclosure causes anti-tumor effects to occur.

This means that SLC1A4-expressing cancers are eligible for an anti-cancer treatment targeting SLC1A4.

As it is shown in the examples herein, a monoclonal antibody directed against the extracellular loop of human SLC1A4 protein of the present disclosure can be used for detecting SLC1A4 at the surface of a variety of SLC1A4 human cancer cells, and thus discriminating between cancer cells expressing SLC1A4 from cancer cells that do not express SLC1A4 at their surface. Any anti-xlSLC1A4 monoclonal antibody of the present disclosure may be used to detect the presence of SLC1A4 at the cell surface of cancer cells, so as to determine whether a subject is affected with a SLC1A4-expressing cancer, or alternatively to stage a SLC1A4-expressing cancer.

The present disclosure also pertains to the *in vitro* use of a monoclonal antibody according to the present disclosure or of an antigen-binding fragment thereof, for detecting SLC1A4 in a tumor sample, preferably in a cell sample.

It relates to an *in vitro* method for detecting the presence of SLC1A4, or SLC1A4-expressing cells, in a sample comprising the steps of:
a) bringing into contact a sample to be tested with an anti-xlSLCIA4 monoclonal antibody of the present disclosure, and
b) detecting the binding of the said monoclonal antibody to SLC1A4.

The present disclosure further concerns an *in vitro* method for determining whether an individual affected with a SLC1A4-expressing cancer comprising the step of determining whether a tumor tissue sample previously obtained from the said individual expresses SLC1A4 protein at the cell surface.

The present disclosure further relates to an *in vitro* method for determining whether an individual affected with a serine-dependent cancer comprising the step of determining whether a tumor tissue sample previously obtained from the said individual expresses SLC1A4 protein at the cell surface.

The present disclosure further pertains to a method for determining whether an individual is affected with a SLC1A4-expressing cancer comprising the steps of:
a) administering an anti-xlSLC1A4 monoclonal antibody of the present disclosure which is labeled with a detectable molecule, and
b) detecting labeling of cancer cells by the said labeled anti-xlSLC1A4 monoclonal antibody, wherein detection of the labeling of cancer cells is indicative of a SLC1A4-expressing cancer.

The above method can be performed through any *in vivo* immuno-imaging technique that is well known in the art.

Is also provided herein a method of imaging a cancer tissue that expresses SLC1A4 in a subject, comprising the steps of:
a) administering to the said subject a labeled anti-xlSLC1A4 monoclonal antibody according to the present disclosure, and
b) visualizing the SLC1A4 expression, such as by radioimmunoscintigraphy or by positron emission tomography (PET).

The present disclosure further pertains to a method for identifying a subject to be suitable for anti-tumor therapy with an anti-xlSLC1A4 monoclonal antibody as disclosed herein comprising the steps of:
a) administering to the said subject a labeled anti-xlSLC1A4 monoclonal antibody according to the present disclosure, and
b) visualizing the SLC1A4 expression, such as by radioimmunoscintigraphy or by positron emission tomography (PET),
wherein the presence of the said labeled anti-xlSLC1A4 monoclonal antibody at a tumor site identifies the subject as suitable for anti-tumor therapy with the said anti-xlSLC1A4 monoclonal antibody.

The present disclosure also relates to a method for monitoring the efficacy of an anti-tumor therapy in a subject, comprising the steps of:
a) selecting a subject with a SLC1A4-expressing tumor wherein the subject is being treated with an anti-tumor therapy,
b) administering a labeled anti-xlSLC1A4 monoclonal antibody according to the present disclosure,
c) imaging the localization of the administered labeled anti-xlSLC1A4 monoclonal antibody, and
d) determining tumor growth,
wherein a decrease from the baseline in uptake of the label signal indicates tumor regression and efficacy of the anti-tumor therapy.

In some embodiments, the said anti-xlSLC1A4 monoclonal antibody is labeled with a positron emitter and visualizing step or imaging step is performed by positron emission tomography (PET).

In embodiments wherein an anti-xlSLC1A4 monoclonal antibody is labeled with a positron emitter, the said antibody is bonded to one or more moieties having the following structure (I) : -L-Mz, wherein (i) L is a chelating moiety, M is a positron emitter and z, independently at each occurrence, is 0 or 1, wherein at least one of z is 1.

In other embodiments, an anti-xlSLC1A4 monoclonal antibody is labeled with a radioactive molecule and visualizing step or imaging step is performed by, e.g., radioimmunoscintigraphy.

Preferably appropriate labeling isotopes can be selected among ¹¹⁰ In, ¹¹¹ In, ¹⁷⁷ Lu, ¹⁸ F, ⁵² Fe, ⁶² Cu, ⁶⁴ Cu, ⁶⁷ Cu, ⁶⁷ Ga, ⁶⁸ Ga, ⁸⁶ Y, ⁹⁰ Y, ⁸⁹ Zr, 94m Tc, 94 Tc, ^{9 9m} Tc, ¹²⁰ I, ¹²³ I, ¹²⁴ I, ¹²⁵ I, ¹³¹ I, ³² P, ¹¹ C, ¹³ N, ¹⁸⁶ Re, ¹⁸⁸ Re, ⁵¹ Mn, ^{52m} Mn, ⁵⁵ Co, ⁷² As, ⁷⁵ Br, ^{7 6} Br, ^{82m} Rb, ⁸³ Sr or any other gamma-, beta- or positron-emitters.

As it is shown in the examples herein, any SLC1A4-expressing cancer, e.g. a melanoma or a liposarcoma, may be treated by an anti-xlSLC1A4 monoclonal antibody of the present disclosure, provided that tumor cells from the cancer tumor express SLC1A4 at their membrane, thus provided that the presence of SLC1A4 proteins at the tumor cell membrane can be detected or determined according to any method.

Thus, the experimental data provided in the examples herein show that the same anti-xlSLC1A4 monoclonal antibody, is effective for treating a plurality of distinct kinds of cancer provided that the SLC1A4 target protein is expressed at the tumor cell membrane.

Incidentally, in the field of anti-cancer active ingredients consisting of target-binding molecules, e.g. target-binding antibodies, the situation wherein the same active ingredient is effective for treating a plurality of distinct cancers is not unprecedented. Illustratively, the anti-PD1 antibody named pembrolizumab has been authorized by the US Food and Drug Administration (FDA) as an active ingredient useful in the treatment of a variety of distinct kinds of cancers, provided that the said cancers share the same physiological features.

Thus, an individual affected with a SLC1A4-expressing cancer may be treated for the said cancer with an anti-xlSLC1A4 monoclonal antibody as described herein when SLC1A4 membrane expression by the tumor cells previously collected from the said individual is detected or otherwise determined by an appropriate method.

The membranous SLC1A4 expression level may be based on the in vitro immuno-histochemical evaluation of the SLC1A4 expression by the cancer cells tested. In vivo immuno-imaging of cancerous patients based on properly labelled anti xlSLC1A4 monoclonal antibody describes herein could also be used to determine the SLC1A4 expression by the tumor.

### Therapeutic uses

The inventors have shown that the anti-xlSLC1A4 monoclonal antibodies of the present disclosure may be advantageously used for treating SLC1A4-expressing cancers.

The inventors have shown herein that the anti-xlSLCIA4 monoclonal antibodies of the present disclosure are useful as novel therapeutic tools for treating SLC1A4-expressing cancers, which consist of serine-dependent cancers.

Thus, the present disclosure relates to an anti-xlSLC1A4 monoclonal antibody of the present description, an antigen-binding fragment thereof, or an immunoconjugate thereof, for use as a drug.

It also concerns the use of an anti-xlSLC1A4 monoclonal antibody of the present disclosure, an antigen-binding fragment thereof, or an immunoconjugate thereof, for preparing a medicament.

The present disclosure also pertains to an anti-xlSLC1A4 of the present disclosure, an antigen-binding fragment thereof, or an immunoconjugate thereof, for its use for treating a SLC1A4-expressing cancer in a subject.

The present disclosure also pertains to the use of an anti-xlSLC1A4 of the present disclosure for the preparation of a medicament for treating a SLC1A4-expressing cancer.

The present disclosure also concerns a method for treating a SLC1A4-expressing cancer comprising a step of administering to an individual in need thereof an anti-xlSLC1A4 monoclonal antibodies of the present disclosure

The present disclosure also concerns a method for treating a SLC1A4-expressing cancer comprising a step of administering to an individual in need thereof a therapeutically effective amount of an anti-xlSLC1A4 monoclonal antibodies of the present disclosure.

The present disclosure further pertains to a method for treating a cancer in a subject comprising the steps of:
a) determining, *in vitro* or *in vivo,* whether the subject's tumor cells express SLC1A4, and
b) administering to the said subject an anti-xlSLC1A4 monoclonal antibody according to the present disclosure, or an immunoconjugate thereof, when SLC1A4 tumor cell expression has been determined at step a).

In some embodiments, step a) is performed *in vitro, e.g.* by providing a tumor sample previously obtained from the subject, *e.g.* a sample originating from a biopsy tumor sample.

In some other embodiments, step a) is performed *in vivo, e.g.* by an immuno-imaging technique, such as described elsewhere in the present disclosure.

The anti-xlSLC1A4 monoclonal antibodies, antigen-binding fragments thereof or immunoconjugates of the present disclosure may be used alone or in combination with any other suitable agent.

The inventors believe, without wishing to be bound by any particular theory, that a single kind of cancer, *e.g.* a lung cancer, may possess a plurality of distinct phenotypes depending on the subject affected therewith. Illustratively, the inventors believe that some lung cancer subjects can be affected with a SLC1A4-expressing lung cancer whereas some other lung cancer subjects can be affected with a lung cancer that does not express SLC1A4. Indeed, sole the subjects who are affected with a SLC1A4-expressing lung cancer are eligible to a therapeutic treatment with an anti-xlSLC1A4 monoclonal antibody of the present disclosure. Otherwise said, the present inventors believe that an anti-xlSLC1A4 monoclonal antibody of the present disclosure can be used for treating a large variety of distinct cancers, the sole provision being that the said cancer expresses SLC1A4.

Examples of SLC1A4-expressing cancers include but are not limited to, melanoma and liposarcoma.

In some embodiments, a SLC1A4-expressing cancer may be selected from the group comprising; bone cancer, brain cancer, ovary cancer, breast cancer, lung cancer, colorectal cancer, osteosarcoma, skin cancer, malignant hemopathies, melanoma, prostate cancer and liposarcoma.

The term "liposarcoma" or "LPS" has its general meaning in the art and refers to soft tissue sarcomas of mesenchymal origin such as revised in the World Health Organisation Classification ICD10 C49.9. The term "liposarcoma" also refers to well-differentiated and dedifferentiated liposarcoma (WD- and DD-LPS). The term "liposarcoma" also relates to Malignant mesenchymal neoplasms, a type of soft tissue sarcoma, a group of lipomatous tumors of varying severity ranging from slow-growing to aggressive and metastatic. Liposarcomas are most often located in the lower extremities or retroperitoneum, but they can also occur in the upper extremities, neck, peritoneal cavity, spermatic cord, breast, vulva and axilla. The term "liposarcoma" also relates to dedifferentiated liposarcoma and well-differentiated liposarcoma.

In an embodiment, the antibodies, fragments thereof and immunoconjugates of the disclosure are particularly suitable for the treatment of, without limitation, the following cancers, provided that these cancers have been determined to consist of SLC1A4-expressing cancers : Acute Lymphoblastic Leukemia (ALL), Acute Myeloid Leukemia (AML), Adrenocortical Carcinoms, Childhood cancers, AIDS-Related Cancers, Kaposi Sarcoma, AIDS-Related Lymphoma, Primary CNS Lymphoma, Anal Cancer, Astrocytomas, Atypical Teratoid/Rhabdoid Tumor, Basal Cell Carcinoma, Skin Cancer (Nonmelanoma), Bile Duct Cancer, Bladder Cancer, Bone Cancer, Ewing Sarcoma Family of Tumors, Osteosarcoma and Malignant Fibrous Histiocytoma, Brain Stem Glioma, Atypical Teratoid/Rhabdoid Tumor, Embryonal Tumors, Germ Cell Tumors, Craniopharyngioma, Ependymoma, Breast Cancer, Bronchial Tumors, Burkitt Lymphoma, Non-Hodgkin Lymphoma, Carcinoid Tumor, Gastrointestinal Carcinoma, Cardiac (Heart) Tumors, Primary Lymphoma, Cervical Cancer, Cholangiocarcinoma, Chordoma, Chronic Lymphocytic Leukemia (CLL), Chronic Myelogenous Leukemia (CML), Chronic Myeloproliferative Neoplasms, Colon Cancer, Colorectal Cancer, Craniopharyngioma, Cutaneous T-Cell Lymphoma, Mycosis Fungoides and Sezary Syndrome, Ductal Carcinoma In Situ (DCIS), Embryonal Tumors, Endometrial Cancer, Ependymoma, Esophageal Cancer, Esthesioneuroblastoma, Extracranial Germ Cell Tumor, Extragonadal Germ Cell Tumor, Eye Cancer, Intraocular Melanoma, Retinoblastoma, Fallopian Tube Cancer, Fibrous Histiocytoma of Bone, Malignant, and Osteosarcoma, Gallbladder Cancer, Gastric (Stomach) Cancer, Gastrointestinal Carcinoid Tumor, Gastrointestinal Stromal Tumors (GIST), Germ Cell Tumor, Ovarian, Testicular, Gestational Trophoblastic Disease, Glioma, Hairy Cell Leukemia, Head and Neck Cancer, Hepatocellular (Liver) Cancer, Histiocytosis, Langerhans Cell, Hodgkin Lymphoma, Hypopharyngeal Cancer, Islet Cell Tumors, Pancreatic Neuroendocrine Tumors, Kaposi Sarcoma, Kidney, Renal Cell, Langerhans Cell Histiocytosis, Laryngeal Cancer, Leukemia, Acute Lymphoblastic (ALL), Acute Myeloid (AML), Chronic Lymphocytic (CLL), Chronic Myelogenous (CML), Hairy Cell, Lip and Oral Cavity Cancer, Liver Cancer (Primary), Lung Cancer, Non-Small Cell, Small Cell, Lymphoma, Hodgkin, Non-Hodgkin, Macroglobulinemia, Waldenstrom, Male Breast Cancer, Melanoma, Merkel Cell Carcinoma, Mesothelioma,Metastatic Squamous Neck Cancer with Occult Primary, Midline Tract Carcinoma Involving NUT Gene, Mouth Cancer, Multiple Endocrine Neoplasia Syndromes, Multiple Myeloma/Plasma Cell Neoplasm, Mycosis Fungoides, Myelodysplasia Syndromes, Myelodysplastic/Myeloproliferative Neoplasms, Myelogenous Leukemia, Chronic (CML), Myeloid Leukemia, Acute (AML) Myeloma, Multiple, Myeloproliferative Neoplasms, Nasal Cavity and Paranasal Sinus Cancer, Nasopharyngeal Cancer, Neuroblastoma, Non-Hodgkin Lymphoma, Non-Small Cell Lung Cancer, Oral Cancer, Oral Cavity Cancer, Lip and Oropharyngeal Cancer, Osteosarcoma and Malignant Fibrous Histiocytoma of Bone, Ovarian Cancer, Low Malignant Potential Tumor, Pancreatic Cancer, Pancreatic Neuroendocrine Tumors (Islet Cell Tumors), Papillomatosis, Paraganglioma, Paranasal Sinus and Nasal Cavity Cancer, Parathyroid Cancer, Penile Cancer, Pharyngeal Cancer, Pheochromocytoma, Pituitary Tumor, Plasma Cell Neoplasm/Multiple Myeloma, Pleuropulmonary Blastoma, Pregnancy and Breast Cancer, Primary Central Nervous System (CNS) Lymphoma, Primary Peritoneal Cancer, Prostate Cancer, Rectal Cancer, Renal Cell (Kidney) Cancer, Renal Pelvis and Ureter, Transitional Cell Cancer, Retinoblastoma, Rhabdomyosarcoma, Salivary Gland Cancer, Rhabdomyosarcoma, Uterine, Small Intestine Cancer, Soft Tissue Sarcoma, Sqamous Cell Carcinoma, Squamous Neck Cancer with Occult Primary, Metastatic, Ttomach (Gastric) Cancer, T-Cell Lymphoma, Testicular Cancer, Throat Cancer, Thymoma and Thymic Carcinoma, Thyroid Cancer, Transitional Cell Cancer of the Renal Pelvis and Ureter, Unknown Primary, Ureter and Renal Pelvis, Transitional Cell Cancer, Urethral Cancer, Uterine Cancer, Endometrial, Uterine Sarcoma, Vaginal Cancer, Vulvar Cancer, Waldenstrom Macroglobulinemia, and Wilms Tumor.

In certain embodiments, an anti-xlSLC1A4 monoclonal antibody of the present disclosure, an antigen-binding fragment thereof, or an immunoconjugate, in particular an antibody-drug conjugate, according to the disclosure is used in combination with a one or more other active agents for preventing or treating a subject affected with a SLC1A4-expressing cancer, which is a serine-dependent cancer.

When used for treating a SLC1A4-expressing cancer, an anti-xlSLC1A4 monoclonal antibody of the present disclosure, an antigen-binding fragment thereof or an immunoconjugate, in particular an ADC, of the present disclosure can be used in combination with conventional cancer therapies such as, e.g., surgery, radiotherapy, chemotherapy, or combinations thereof.

In certain aspects, other therapeutic agents useful for combination cancer therapy with an anti-xlSLC1A4 monoclonal antibody of the present disclosure, an antigen-binding fragment thereof or and antibody-drug conjugate in accordance with the present disclosure, can include one or more other active agents aimed at depriving the SLC1A4-expressing tumor cells from serine.

An active agent causing deprivation of SLC1A4-expressing tumor cells from serine encompass inhibitors of the Interleukin-6 (IL-6) signaling pathway. Inhibitors of the Interleukin-6 (IL-6) signaling pathway can be selected from an IL-6 inhibitor, an IL-6 receptor inhibitor, an IL-6/IL-6 receptor complex inhibitor, a gp130 inhibitor and a STAT3 inhibitor.

The term "Interleukin-6 (IL-6) signaling inhibitor" refers to compounds or agents that selectively block or inactivate the IL-6 signaling pathway. As used herein, the term "selectively blocks or inactivates" refers to a compound that preferentially binds to and blocks or inactivate IL-6, IL-6 receptor, IL-6/IL-6R complex, STAT3 and/or gp130. Especially compounds that block the interaction between IL-6 with its IL-6 receptor, IL-6 with gp130, IL-6 receptor with gp130, IL-6/IL-6 Receptor complex with gp130, or compounds that blocks the phosphorylation of STAT3. Typically, an IL-6 signaling inhibitor may encompass without limitation a polypeptide, an aptamer, an antibody or a portion thereof, an antisense oligonucleotide, *i.e.,* a siRNA or a shRNA, or a ribozyme.

The terms "IL-6/IL-6R complex" or "IL-6/IL-6 receptor complex" collectively refer to a complex formed by a soluble form of IL-6 and membrane-bound form IL-6 receptor alpha. In some embodiments, "IL-6/IL-6R complex" refers to a complex formed by (i) a soluble form of IL-6 secreted by a cancer cell of a subject, and (ii) a membrane-bound form IL-6 receptor alpha of a myoblast cell of the same subject.

In some embodiments, the IL-6 signaling inhibitor that may be used in combination with an anti-xlSLC1A4 monoclonal antibody of the present disclosure can be itself an antibody, or an antigen-binding portion thereof, directed against one of IL-6, IL-6 receptor, STAT 3 and/or gp130.

In some other embodiments, the IL-6 signaling inhibitor that can be used in combination with an anti-xlSLC1A4 monoclonal antibody of the present disclosure may be an IL-6 expression inhibitor, an IL-6 receptor expression inhibitor, a STAT 3 expression inhibitor and/or a gp130 expression inhibitor.

In some embodiments, expression inhibitors for use in the method according to the present disclosure may be anti-sense oligonucleotides.

In some embodiments, the IL-6 signaling inhibitor of the present disclosure may be a chemical compound.

In some embodiments, a chemical compound of the present disclosure includes compounds inhibiting the IL-6 expression, IL-6R expression, STAT expression, the phosphorylation of STAT3 and/or gp 130 expression.

In some embodiments, chemical compounds that can be used in combination with an anti-xlSLC1A4 monoclonal antibody of the present disclosure may be blocking anti-gp130 compounds, compounds that inhibit the phosphorylation of STAT3 or compounds that inhibit the expression of STAT3.

Some of those blocking anti-gp130 compound are illustrated by, for instance, Wu et al., Mol Cancer Ther. 2016 Nov; 15(11):2609-2619.

In some embodiments, a gp-130 inhibitor of the present disclosure may be bazedoxifene,

In some embodiments, a STAT3 inhibitor may be selected from SH5-07, APTSTAT3-9R, C188-9, BP-1-102, Niclosamide (BAY2353), STAT3-IN-1, WP1066, Cryptotanshinone, Stattic, Resveratrol (SRT501), S31-201, HO-3867, Napabucasin (BBI608), Brevilin A, Artesunate (WR-256283), Bosutinib (SKI-606), TPCA-1, SC-43, Ginkgolic acid C17:1, Ochromycinone (STA-21), Colivelin, Cucurbitacin IIb, GYY4137, Scutellarin, Kaempferol-3-O-rutinoside, Cucurbitacin I, SH-4-54, Nifuroxazide and Pimozide.

In some embodiments, a STAT3 inhibitor may be C188-9, Stattic or a combination thereof.

As used herein "bazedoxifene" has its general meaning in the art and consists of a gp130 inhibitor having the formula: 1-[[4-[2-(Hexahydro-1H-azepin-1-yl)ethoxy]phenyl]methyl]-2-(4-hydroxyphenyl)-3-methyl-1H-indol-5-ol monoacetate (salt) with the molecular formula C30H34N2O3 • C2H4O2 and the CAS Number 198481-33-3.

As used herein "C188-9" is a potent inhibitor of STAT3 that binds to STAT3 with high affinity. C188-9 may be defined by the reference: CAS No. 432001-19-9.

As used herein "Stattic" is a small molecule inhibiting STAT3 activation. Stattic may be defined by the reference: CAS No. 19983-44-9.

### Pharmaceutical compositions

For administration, the anti-xlSLC1A4 monoclonal antibody of the present disclosure, an antigen-binding fragment(s) thereof or antibody-drug conjugate is preferably formulated as a pharmaceutical composition.

A pharmaceutical composition comprising an anti-xlSLC1A4 monoclonal antibody of the present disclosure, an antigen-binding fragment thereof, or an antibody-drug conjugate can be formulated according to known methods to prepare pharmaceutically useful compositions, whereby the therapeutic molecule is combined in a mixture with a pharmaceutically acceptable carrier.

A composition is said to be a "pharmaceutically acceptable carrier" if its administration can be tolerated by a recipient patient. Sterile phosphate-buffered saline is one example of a pharmaceutically acceptable carrier. Other suitable carriers are well-known to those in the art. (See, e.g., Gennaro (ed.), Remington's Pharmaceutical Sciences (Mack Publishing Company, 19th ed. 1995).) Formulations may further include one or more excipients, preservatives, solubilizers, buffering agents, albumin to prevent protein loss on vial surfaces, etc.

### Routes of administration

The form of the pharmaceutical compositions, the route of administration, the dosage and the regimen naturally depend upon the condition to be treated, the severity of the illness, the age, weight, and sex of the patient, etc.

The pharmaceutical compositions of the disclosure can be formulated for a topical, oral, parenteral, intranasal, intravenous, intramuscular, subcutaneous or intraocular administration and the like.

Preferably, the pharmaceutical compositions contain vehicle(s)/carrier(s) which is/are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The doses used for the administration can be adapted as a function of various parameters, and in particular as a function of the mode of administration used, of the relevant pathology, or alternatively of the desired duration of treatment.

To prepare pharmaceutical compositions, an effective amount of the antibody may be dissolved or dispersed in a pharmaceutically acceptable carrier or aqueous medium.

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions of the active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

### Pharmaceutical carrier substances

An anti-xlSLC1A4 monoclonal antibody of the present disclosure can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

The preparation of more, or highly concentrated solutions for direct injection is also contemplated, where the use of DMSO as solvent is envisioned to result in extremely rapid penetration, delivering high concentrations of the active agents to a small tumor area.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject.

The anti-xlSLC1A4 monoclonal antibodies of the present disclosure may be formulated within a therapeutic mixture so as to be adapted for administration of the monoclonal antibodies at a dose range of from about 0.1 mg to about 10 mg per kilogram of body weight, advantageously at a dose range of from about 0.5 mg to about 5 mg per kilogram of body weight, such as at dose of about 1 mg per kilogram of body weight.

Thus, the anti-xlSLC1A4 monoclonal antibodies of the present disclosure may be formulated within a therapeutic mixture that can comprise from about 8 milligrams to 800 milligrams, advantageously from about 4 milligrams to 400 milligrams, such as about 80 milligrams per dose or so. Multiple doses can also be administered.

In addition to the compounds formulated for parenteral administration, such as intravenous or intramuscular injection, other pharmaceutically acceptable forms include, e.g. tablets or other solids for oral administration; time release capsules ; and any other form currently used.

In certain embodiments, the use of liposomes and/or nanoparticles is contemplated for the introduction of antibodies into host cells. The formation and use of liposomes and/or nanoparticles are known to those of skill in the art.

Nanocapsules can generally entrap compounds in a stable and reproducible way. To avoid side effects due to intracellular polymeric overloading, such ultrafine particles (sized around 0.1 µm) are generally designed using polymers able to be degraded in vivo. Biodegradable polyalkyl-cyanoacrylate nanoparticles that meet these requirements are contemplated for use in the present disclsoure, and such particles may be are easily made.

Liposomes are formed from phospholipids that are dispersed in an aqueous medium and spontaneously form multilamellar concentric bilayer vesicles (also termed multilamellar vesicles (MLVs)). MLVs generally have diameters of from 25 nm to 4 µm. Sonication of MLVs results in the formation of small unilamellar vesicles (SUVs) with diameters in the range of 200 to 500 Å, containing an aqueous solution in the core. The physical characteristics of liposomes depend on pH, ionic strength and the presence of divalent cations.

The description will be further illustrated by the following figures and examples. However, these examples and figures should not be interpreted in any way as limiting the scope of the present disclosure.

### EXAMPLES

### A. MATERIALS AND METHODS

### A.1. Cell culture:

Three cell lines were used, expressing different level of the SLCA14 transporter, respectively (i) Human DD-liposarcoma IB115, (ii) human melanoma SKMEL 5 and (iii) human pancreatic cancer HPAC.

The cell lines were cultured (i) in DMEM (for IB 115 and SKMEL 5 ) or in (ii) DMEM-F12 (for HPAC) medium with 10% decomplemented fetal calf serum and 1% PenStrep (streptomycin and penicillin). In addition, IB115 were transduced with shSLC1A4 (short hairpin nucleic acid directed to SLC1A4 mRNA) to verify antibody specificity. The shRNA is of the nucleic acid sequence of SEQID NO. 54 and targets the nucleic acid sequence of SEQ ID NO. 53.

To determine therapeutical effect of antibodies on cells, 2000 IB115 cells were plated in 96 wells plate (day 0). 50µg/ml of antibodies were added with physiological medium (EMEM, 1% dialyzed serum, comprising the following seven non essential amino acids: Proline, alanine, asparagine, aspartate, glutamate, glycine, serine) at day 1 and 3. Confluency was observed and analyzed using Incucyte Systems for Live-Cell Imaging and Analysis. After 7 days, sulforhodamine coloration was done and DO was measured at 560nm.

### A.2. Western blot assay

The Western blot assay was performed as disclosed hereunder.
- 2µg of antibody are loaded per well (condition non reduced : 2µg+Laemli ; condition reduced : 2µg+Laemli+Bmercapto-ethanol, boiled 10 min 95°C)
- gel migration is done for 1hour at 160V in precast gel, Gebagel 4-12% (ref 15G-0412-10 GeneBio Apllication)

After migration, gel is colored by Gelcode blue safe Protein stain (ref 1860957 Thermo Scientific) and washed with water.

### A.3. Quantitative RT-PCR:

The RNA samples were deposited on 384-well plates and analyzed by the LightCycler 480^{©}. The programmed protocol consisted of a 10-minute pre-incubation phase at 95°C for polymerase activation followed by 45 cycles of amplification. Quantitative analysis of gene expression was determined by the ΔΔCt method and the β-actine gene was used as a reference for normalization.

### A.4. PDX Mice

Swiss nude mice were engrafted with human dedifferentiated 20 LPS fragments (20-30mm2) subcutaneously.

When tumors reached 100 mm3, mice were treated bi-weekly with 10 mg/kg antibody by intraperitoneal injection. Tumors were measured bi-weekly using a caliper.

### A.5. Fluorescence Activated Cell Sorting (FACS)

300 000 cells were put in 96 R wells plate and fixed with 4% paraformaldehyde 15 min at room temperature. We incubated cells with primary antibodies diluted in PBS-BSA 2% during 1h at RT. Secondary antibody was used at 1/100 and incubate on cells during 1h at RT. Positive cells were measured by CytoFLEX^{®} Flow Cytometry (Beckman) and results analysed with FlowJo^{®} software.

### Example 1: Binding of monoclonal anti-SLC1A4 to SLC1A4-expressing cells

### 1.A. SLC1A4 protein and mRNA expression by various human cancer cell lines

A plurality of human cancer cell lines were tested for their ability to express SLC1A4 target protein, namely (i) SK-MEL-5 melanoma cell line; (ii) SK-MEL-28 melanoma cell line, (iii) HPAC pancreatic cancer cell line, (iv) A2058 melanoma cell line, (v) MW2664 melanoma cell line and (vi) IB 115 sarcoma cell line.

The results are shown in figure 1 (figures 1A and 1B). Figure 1A shows that all the tested cancer cell lines express SLC1A4, except the HPAC pancreatic cell line.

The results depicted in Figure 1B show that SLC1A4 mRNA expression was substantially higher in SK-MEL-5 melanoma cell line that in IB115 liposarcoma cell line, whereas no SLC1A4 mRNA expression was detected in the HPAC pancreatic cancer cell line.

### 1.B. Production of monoclonal antibodies directed against the external loop of SLC1A4

The binding of the monoclonal antibodies of this disclosure to the external loop of the SLC1A4 protein is schematically represented in Figure 2A.

Effective production of anti-SLC1A4 monoclonal antibodies was verified by Western blotting. For each of the B5.1, E10.2 D6.2, G11.4, B8.3 and A10 antibodies, it was ensured that an effective production of monoclonal antibodies comprising (i) both the heavy and light chains in associated form (non-reducing conditions) or (ii) dissociated into each of the heavy in light chains (reducing conditions, as it shown on Figure 2B.

### 1.C. Binding of anti-SLC1A4 monoclonal antibodies to human cancer cells.

Binding of anti-SLC1A4 monoclonal antibodies (with their human Fc fragment replaced by a rabbit Fc domain) to various cancer cell lines was assayed, namely the B5.1, E10.2 D6.2, G11.4, B8.3 and A10 monoclonal antibodies.

As it is shown in Figure 3, each of the tested antibodies bound to the SLC1A4-expressing cancer cells. As it was expected, none of the anti-SLC1A4 antibodies bound to the HPAC pancreatic cells that do not express SLC1A4.

The same results were obtained with the human anti-SLC1A4 monoclonal antibodies having the same variable regions and a human IgG 1 Fc domain. The results of Figure 4 show that the fully human anti-SLC1A4 monoclonal antibodies E10, D6, B8 and G11 bound to the SLC1A4-expressing melanoma cell line SK-MEL-5, while no detectable binding occurred to the HPAC pancreatic cells that do not express SLC1A4.

### Example 2 : In vitro anti-proliferative effect of the monoclonal anti-SLC1A4 antibodies

The *in vitro* anti-tumor effect of human anti-SLC1A4 monoclonal antibodies anti-SLC1A4 monoclonal antibodies B5, A10, B8, D6, E10 and G11 engineered with a rabbit Fc was assayed on the human liposarcoma IB115 cell line.

The results show that all of the tested anti-SLC1A4 monoclonal antibodies possess a significant anti-proliferative effect against the human sarcoma cells.

The *in vitro* anti-tumor effect of the fully human anti-SLC1A4 monoclonal antibodies B8, D6, G11 and E10 was assayed on the human liposarcoma IB115 cell line.

The results are depicted in Figure 5, wherein the tumor cell killing capacity of the human monoclonal antibodies B8, D6, E10 and G11 was assayed.

The results of Figure 5 show that the anti-SLC1A4 human monoclonal antibodies of this disclosure possess a high capacity of killing tumor cells.

### Example 3 : In vivo anti-tumor properties if the anti-SLC1A4 human monoclonal antibodies

The *in vivo* anti-tumor properties of the human anti-SLC1A4 monoclonal antibodies were assayed in PDX mice that were xenografted with human dedifferentiated liposarcoma cells derived from a patient affected with liposarcoma.

The results show that all the anti-SLC1A4 human monoclonal antibodies tested, namely B8, D6, E10 and G11, are able to reduce the tumor growth.

The results show, for each of the tested antibodies, a reduction of the tumor growth occurring with time.

| **TABLE OF SEQUENCES** | | |
|---|---|---|
| **SEQ ID NO.** | **Sequence** | **Comme nt** |
| ***B5 mAb*** | | |
| **1** | GFTFSNYY | VH-CDR1 |
| **2** | YIYGSSRD | VH-CDR2 |
| **3** | VRSSNSGGMDV | VH-CDR3 |
| **4** | MAEVQLVESGGSLVKPGGSLRLSCAASGFTFSNYYMNWVR QAPGKGLE WISYIYGSSRDIGYADFVKGRFTISRDNAKNSLYLQMNSLRA EDTAVYYC VRSSNSGGMDVWGRGTLV | VH |
| **5** | SSDVGGGSD | VL-CDR1 |
| **6** | GDS | VL-CDR2 |
| **7** | SSYTYYSTRV | VL-CDR3 |
| **8** | TVSSGGGGSGGGGSGGGGSQSVLTQPASVSGSPGQSITISCA GTSSDVG GGSDVSWYQQHPGKAPKLMIYGDSYRPSGVSNRFSGSKSG NTASLTISGL QAEDEADYYCSSYTYYSTRVFGGGTKL | VL |
| | | |
| ***D6 mAb*** | | |
| **9** | GFTFSNYY | VH-CDR1 |
| **10** | SIYGSSRG | VH-CDR2 |
| **11** | VRSNYGMDV | VH-CDR3 |
| **12** | MAEVQLVESGGSLVKPGGSLRLSCAASGFTFSNYYMNWVR QAPGKGLEWISSIYGSSRGIGYADFVKGRFTISRDNAKNSLY LQMNSLRAEDTAVYYCVRSNYGMDVWGRGTLVTV | VH |
| **13** | SSDVGGYSD | VL-CDR1 |
| **14** | SDS | VL-CDR2 |
| **15** | SSSTDYSTR | VL-CDR3 |
| **16** | SSGGGGSGGGGSGGGGSQSVLTQPASVSGSPGQSITISCAGT SSDVGGYSDVSWYQQHPGKAPKLMIYSDSQRPSGVSNRFSG SKSGNTASLTISGLQAEDEADYYCSSSTDYSTRVFGGGTKL | VL |
| | | |
| ***A10 mAb*** | | |
| **17** | GFTFSNAY | VH-CDR1 |
| **18** | GIYGSSRS | VH-CDR2 |
| **19** | VRSSSSYGSGMD | VH-CDR3 |
| **20** | MAEVQLVESGGSLVKPGGSLRLSCAASGFTFSNAYMNWVR QAPGKGLEWISGIYGSSRSIYYADFVKGRFTISRDNAKNSLY LQMNSLRAEDTAVYYCVRSSSSYGSGMDVWGRGTL | VH |
| **21** | SSDVGGSDD | VL-CDR1 |
| **22** | GDS | VL-CDR2 |
| **23** | SSNTYDSTRV | VL-CDR3 |
| **24** | VTVSSGGGGSGGGGSGGGGSQSVLTQPASVSGSPGQSITISC AGTSSDVGGSDDVSWYQQHPGKAPKLMIYGDSNRPSGVSN RFSGSKSGNTASLTISGLQAEDEADYYCSSNTYDSTRVFGGG TKL | VL |
| | | |
| ***E10 mAb*** | | |
| **25** | GFTFSNAA | VH-CDR1 |
| **26** | NIQGSSSS | VH-CDR2 |
| **27** | VRSSSSYGSGMDV | VH-CDR3 |
| **28** | MAEVQLVESGGSLVKPGGSLRLSCAASGFTFSNAAMNWVR QAPGKGLEWISNIQGSSSSIYYADFVKGRFTISRDNAKNSLY LQMNSLRAEDTAVYYCVRSSDDSFGSGMDVWGRG | VH |
| **29** | SSDVGGYYS | VL-CDR1 |
| **30** | DDS | VL-CDR2 |
| **31** | SSYTSYSTRV | VL-CDR3 |
| **32** | TLVTVSSGGGGSGGGGSGGGGSQSVLTQPASVSGSPGQSITI SCAGTSSDVGGYYSVSWYQQHPGKAPKLMIYDDSNRPSGV SNRFSGSKSGNTASLTISGLQAEDEADYYCSSYTSYSTRVFG GGTKL | VL |
| | | |
| ***G11 mAb*** | | |
| **33** | GFTFSNYG | VH-CDR1 |
| **34** | SISGSSSS | VH-CDR2 |
| **35** | VRSYYGMDV | VH-CDR3 |
| **36** | MAEVQLVESGGSLVKPGGSLRLSCAASGFTFSNYGMNWVR QAPGKGLEWISSISGSSSSISYADFVKGRFTISRDNAKNSLYL QMNSLRAEDTAVYYCVRSYYGMDVWGRGTLVTVS | VH |
| **37** | SSDVGGYGQ | VL-CDR1 |
| **38** | SDS | VL-CDR2 |
| **39** | SSYTQDSTRV | VL-CDR3 |
| **40** | SGGGGSGGGGSGGGGSQSVLTQPASVSGSPGQSITISCAGTS SDVGGYGQVSWYQQHPGKAPKLMIYSDSSRPSGVSNRFSGS KSGNTASLTISGLQAEDEADYYCSSYTQDSTRVFGGGTKL | VL |
| | | |
| ***B8 mAb*** | | |
| **41** | GFTFSNNA | VH-CDR1 |
| **42** | YISGSSRN | VH-CDR2 |
| **43** | VRSSYYGFGSGMDV | VH-CDR3 |
| **44** | MAEVQLVESGGSLVKPGGSLRLSCAASGFTFSNNAMNWVR QAPGKGLEWISYISGSSRNIYYADFVKGRFTISRDNAKNSLY LQMNSLRAEDTAVYYCVRSSYYGFGSGMDVWGRG | VH |
| **45** | SSDVGGNSY | VL-CDR1 |
| **46** | YDS | VL-CDR2 |
| **47** | SSNTGYSTRV | VL-CDR3 |
| **48** | TLVTVSSGGGGSGGGGSGGGGSQSVLTQPASVSGSPGQSITI SCAGTSSDVGGNSYVSWYQQHPGKAPKLMIYYDSQRPSGV SNRFSGSKSGNTASLTISGLQAEDEADYYCSSNTGYSTRVFG GGTKL | VL |
| | | |
| ***Fc region*** | | |
| **49** | APSTCSKPTCPPPELLGGPSVFIFPPKPKDTLMISRTPEVTCVV VDVSEDDPEVQFTWYI NNEQVRTARPPLREQQFNSTIRVVSTLPIAHEDWLRGKEFKC KVHNKALPAPIEKTISKA RGQPLEPKVYTMGPPREELSSRSVSLTCMINGFYPSDISVEW EKNGKAEDNYKTTPAVLD SDGSYFLYSKLSVPTSEWQRGDVFTCSVMHEALHNHYTQKS ISRSPGK* | Rabbit IgG |
| | | |
| ***Fc region*** | | |
| **50** | EPKSCDKTHTCPPCPAPELLGGPSVFLFPPKPKDTLMISRTPE VTCVVVDVSHEDPEVKF NWYVDGVEVHNAKTKPREEQYNSTYRVVSVLTVLHQDWL NGKEYKCKVSNKALPAPIEKT ISKAKGQPREPQVYTLPPSRDELTKNQVSLTCLVKGFYPSDI AVEWESNGQPENNYKTTP PVLDSDGSFFLYSKLTVDKSRWQQGNVFSCSVMHEALHNH YTQKSLSLSPGK* | Human IgG 1 |
| | | |
| ***SLC1A4*** | | |
| **51** | KPGSGAQTLQSSDLGLEDSGPPPVPKETVDSFLDLARNLFPS NLVVAAFRTYATDYKVVTQNSSSGNVTHEKIPIGT | Human Extracel lular Loop |
| | | |
| **52** | KPGAGAQTLQSSSLGLENSGPPPVSKETVDSFLDLLRNLFPS NLVVAAFTTSATDYTVVTHNTSSGNVTKEKIPVVT | Mouse Extracel lular Loop |
| **53** | CCATGTTATTCATGGAGGAAT | Target sequenc e of shRNA TRC000 0038639 (Sigma) |
| **54** | CCGG-CCATGTTATTCATGGAGGAAT-CTCGAG- ATTCCTCCATGAATAACATGGTTTTTG | shRNA TRC000 0038639 (Sigma) |
| **55** | CCGGCCATGTTATTCATGGAGGAATCTCGAGATTCCT CCATGAATAACATGGTTTTTG | Oligo design for arrayed cloning Forward sequenc e |
| **56** | AATTCAAAAACCATGTTATTCATGGAGGAATCTCGA GATTCCTCCATGAATAACATGG | Oligo design for arrayed cloning Reverse sequenc e |

In case of any discrepancy between the above list of sequences and sequences disclosed in an appended sequence listing (e.g. according to the WIPO Standard ST.26), the correct sequences are those disclosed in the above table included in the present disclosure.

## Claims

1. A monoclonal antibody directed against the extracellular loop of human SLC1A4 protein, wherein the said monoclonal antibody is selected from :
**a)** a monoclonal antibody, or an antigen-binding fragment thereof, comprising :
**(i)** a heavy chain wherein the variable domain comprises :
- a VH-CDR1 having a sequence set forth as SEQ ID NO. 1;
- a VH-CDR2 having a sequence set forth as SEQ ID NO. 2; and
- a VH-CDR3 having a sequence set forth as SEQ ID NO. 3;
and/or
**(ii)** a light chain wherein the variable domain comprises :
- a VL-CDR1 having a sequence set forth as SEQ ID NO. 5;
- a VL-CDR2 having a sequence set forth as SEQ ID NO. 6; and
- a VL-CDR3 having a sequence set forth as SEQ ID NO. 7.
**b)** a monoclonal antibody, or an antigen-binding fragment thereof, comprising :
**(i)** a heavy chain wherein the variable domain comprises :
- a VH-CDR1 having a sequence set forth as SEQ ID NO. 9;
- a VH-CDR2 having a sequence set forth as SEQ ID NO. 10; and
- a VH-CDR9 having a sequence set forth as SEQ ID NO. 11;
and/or
**(ii)** a light chain wherein the variable domain comprises :
- a VL-CDR1 having a sequence set forth as SEQ ID NO. 13;
- a VL-CDR2 having a sequence set forth as SEQ ID NO. 14; and
- a VL-CDR3 having a sequence set forth as SEQ ID NO. 15.
**c)** a monoclonal antibody, or an antigen-binding fragment thereof, comprising :
**(i)** a heavy chain wherein the variable domain comprises :
- a VH-CDR1 having a sequence set forth as SEQ ID NO. 17;
- a VH-CDR2 having a sequence set forth as SEQ ID NO. 18; and
- a VH-CDR9 having a sequence set forth as SEQ ID NO. 19;
and/or
**(ii)** a light chain wherein the variable domain comprises :
- a VL-CDR1 having a sequence set forth as SEQ ID NO. 21;
- a VL-CDR2 having a sequence set forth as SEQ ID NO. 22; and
- a VL-CDR3 having a sequence set forth as SEQ ID NO. 23.
**d)** a monoclonal antibody, or an antigen-binding fragment thereof, comprising :
**(i)** a heavy chain wherein the variable domain comprises :
- a VH-CDR1 having a sequence set forth as SEQ ID NO. 25;
- a VH-CDR2 having a sequence set forth as SEQ ID NO. 26; and
- a VH-CDR9 having a sequence set forth as SEQ ID NO. 27;
and/or
**(ii)** a light chain wherein the variable domain comprises :
- a VL-CDR1 having a sequence set forth as SEQ ID NO. 29;
- a VL-CDR2 having a sequence set forth as SEQ ID NO. 30; and
- a VL-CDR3 having a sequence set forth as SEQ ID NO. 31.
**e)** a monoclonal antibody, or an antigen-binding fragment thereof, comprising :
**(i)** a heavy chain wherein the variable domain comprises :
- a VH-CDR1 having a sequence set forth as SEQ ID NO. 33;
- a VH-CDR2 having a sequence set forth as SEQ ID NO. 34; and
- a VH-CDR9 having a sequence set forth as SEQ ID NO. 35;
and/or
**(ii)** a light chain wherein the variable domain comprises :
- a VL-CDR1 having a sequence set forth as SEQ ID NO. 37;
- a VL-CDR2 having a sequence set forth as SEQ ID NO. 38; and
- a VL-CDR3 having a sequence set forth as SEQ ID NO. 39.
**f)** a monoclonal antibody, or an antigen-binding fragment thereof, comprising :
**(i)** a heavy chain wherein the variable domain comprises :
- a VH-CDR1 having a sequence set forth as SEQ ID NO. 41;
- a VH-CDR2 having a sequence set forth as SEQ ID NO. 42; and
- a VH-CDR9 having a sequence set forth as SEQ ID NO. 43;
and/or
**(ii)** a light chain wherein the variable domain comprises :
- a VL-CDR1 having a sequence set forth as SEQ ID NO. 45;
- a VL-CDR2 having a sequence set forth as SEQ ID NO. 46; and
- a VL-CDR3 having a sequence set forth as SEQ ID NO. 47.

2. The monoclonal antibody **a)** according to claim 1, comprising :
**(i)** a heavy chain variable domain having at least 85% amino acid identity with the heavy chain variable domain of SEQ ID NO. 4 and comprising a VH-CDR1 of SEQ ID NO. 1, a VH-CDR2 of SEQ ID NO. 2 and a VH-CDR3 of SEQ ID NO. 3;
and/or
**(ii)** a light chain variable domain having at least 85% amino acid identity with the light chain variable domain of SEQ ID NO. 8 and comprising a VL-CDR1 of SEQ ID NO. 5, a VL-CDR2 of SEQ ID NO. 6 and a VL-CDR3 of SEQ ID NO. 7.

3. The monoclonal antibody **b)** according to claim 1, comprising :
**(i)** a heavy chain variable domain having at least 85% amino acid identity with the heavy chain variable domain of SEQ ID NO. 12 and comprising a VH-CDR1 of SEQ ID NO. 9, a VH-CDR2 of SEQ ID NO. 10 and a VH-CDR3 of SEQ ID NO. 11;
and/or
**(ii)** a light chain variable domain having at least 85% amino acid identity with the light chain variable domain of SEQ ID NO. 16 and comprising a VL-CDR1 of SEQ ID NO. 13, a VL-CDR2 of SEQ ID NO. 14 and a VL-CDR3 of SEQ ID NO. 15.

4. The monoclonal antibody **c)** according to claim 1, comprising :
**(i)** a heavy chain variable domain having at least 85% amino acid identity with the heavy chain variable domain of SEQ ID NO. 20 and comprising a VH-CDR1 of SEQ ID NO. 17, a VH-CDR2 of SEQ ID NO. 18 and a VH-CDR3 of SEQ ID NO. 19;
and/or
**(ii)** a light chain variable domain having at least 85% amino acid identity with the light chain variable domain of SEQ ID NO. 24 and comprising a VL-CDR1 of SEQ ID NO. 21, a VL-CDR2 of SEQ ID NO. 22 and a VL-CDR3 of SEQ ID NO. 23.

5. The monoclonal antibody **d)** according to claim 1, comprising :
**(i)** a heavy chain variable domain having at least 85% amino acid identity with the heavy chain variable domain of SEQ ID NO. 28 and comprising a VH-CDR1 of SEQ ID NO. 25, a VH-CDR2 of SEQ ID NO. 26 and a VH-CDR3 of SEQ ID NO. 27;
and/or
**(ii)** a light chain variable domain having at least 85% amino acid identity with the light chain variable domain of SEQ ID NO. 32 and comprising a VL-CDR1 of SEQ ID NO. 29, a VL-CDR2 of SEQ ID NO. 30 and a VL-CDR3 of SEQ ID NO. 31.

6. The monoclonal antibody **e)** according to claim 1, comprising :
**(i)** a heavy chain variable domain having at least 85% amino acid identity with the heavy chain variable domain of SEQ ID NO. 36 and comprising a VH-CDR1 of SEQ ID NO. 33, a VH-CDR2 of SEQ ID NO. 34 and a VH-CDR3 of SEQ ID NO. 35;
and/or
**(ii)** a light chain variable domain having at least 85% amino acid identity with the light chain variable domain of SEQ ID NO. 40 and comprising a VL-CDR1 of SEQ ID NO. 37, a VL-CDR2 of SEQ ID NO. 38 and a VL-CDR3 of SEQ ID NO. 39;

7. The monoclonal antibody **e)** according to claim 1, comprising :
**(i)** a heavy chain variable domain having at least 85% amino acid identity with the heavy chain variable domain of SEQ ID NO. 44 and comprising a VH-CDR1 of SEQ ID NO. 41, a VH-CDR2 of SEQ ID NO. 42 and a VH-CDR3 of SEQ ID NO. 43;
and/or
**(ii)** a light chain variable domain having at least 85% amino acid identity with the light chain variable domain of SEQ ID NO. 48 and comprising a VL-CDR1 of SEQ ID NO. 45, a VL-CDR2 of SEQ ID NO. 46 and a VL-CDR3 of SEQ ID NO. 47.

8. The monoclonal antibody according to any one of claims 1 to 8, which is a human monoclonal antibody.

9. An antigen-binding fragment of an antibody according to any one of claims 1 to 8.

10. An immunoconjugate comprising the antibody according to any one of claims 1 to 8 linked to a therapeutic agent, preferably to a cytotoxic agent.

11. An immunoconjugate comprising the antibody according to any one of claims 1 to 8 linked to a detectable molecule.

12. The *in vitro* use of a monoclonal antibody according to any one of claims 1 to 8 or of an antigen-binding fragment thereof according to claim 9, for detecting SLC1A4, or SLC1A4-expressiong cells, in a sample, preferably in a cell sample.

13. A pharmaceutical composition comprising an antibody according to any of claims 1 to 8, or an antigen-binding fragment thereof according to claim 9, or an immunoconjugate according to claim 10, in combination with a pharmaceutically acceptable carrier.

14. The antibody according to any of claims 1 to 8, an antigen-binding fragment thereof according to claim 9, or an immunoconjugate according to claim 10, for use as a drug.

15. An antibody according to any of claims 1 to 8, an antigen-binding fragment thereof according to claim 9, an immunoconjugate according to claim 10, or a pharmaceutical composition according to claim 13s for use for treating a SLC1A4-expressing cancer.
